# FASCICULE DE BREVET EUROPEEN

(11) **EP 1 228 212 B1**
(45) Date de publication et mention de la délivrance du brevet: **06.01.2010**
(21) Numéro de dépôt: 00981414.6
(22) Date de dépôt: 10.11.2000
(51) Int. Cl.: C12N 15/12, C07K 14/705, C07K 16/28, G01N 33/569, A61K 39/395, A61K 35/14, A61K 35/28, C12Q 1/68

(54) **PROTEINE PRESENTE A LA SURFACE DES CELLULES SOUCHES HEMATOPOIETIQUES DE LA LIGNEE LYMPHOIDE ET DES CELLULES NK, ET SES APPLICATIONS.**
PROTEINE, DAS AUF HÄMATOPOIETISCHEN STAMMZELLEN DER LYMPHOID UND NK LINIEN ZU FINDEN IST, UND VERWENDUNGEN DAVON
PROTEIN PRESENT AT THE SURFACE OF HEMATOPOIETIC STEM CELLS OF THE LYMPHOID LINE AND OF NK CELLS, AND USES THEREOF

(30) Priorité: 12.11.1999 FR 9914241
(43) Date de publication de la demande: 07.08.2002
(73) Titulaire: COMMISSARIAT A L'ENERGIE ATOMIQUE, 75015 Paris (FR)
(72) Inventeur: KIRSZENBAUM, Marek, F-75005 Paris (FR); LE DISCORDE, Magali, F-75011 Paris (FR); PROST, Stéphane, F-92290 CHATENAY-MALABRY (FR)
(74) Mandataire: Vialle-Presles, Marie José
(86) Numéro de dépôt international: PCT/FR2000/003137
(87) Numéro de publication internationale: WO 2001/034653

(56) Documents cités:
- WO-A-00/09552
- WO-A-95/06247
- WO-A-95/20604
- WO-A-98/25959
- WO-A-98/39448
- WO-A-99/31236
- DATABASE SWALL EBI, Hinxton, U.K.; Accession Number: Q9Y3U9, 1 novembre 1999 (1999-11-01) KOEHRER K ET AL: "Hypothetical 34.9kDa protein" XP002147605 cité dans la demande
- DATABASE EMEST EBI, Hinxton, U.K.; Accession Number: AW106427, 21 octobre 1999 (1999-10-21) MARRA M ET AL: "Mus musculus cDNA clone IMAGE:2225799 5' similar to WP:Y53C12A.3 CE14888" XP002147606 cité dans la demande

### LISTE DE SEQUENCES

<110> COMMISSARIAT A L'ENERGIE ATOMIQUE
   KIRSZENBAUM, Marek
   LE DISCORDE, Magali
   PROST, Stéphane
<120> PROTEINE PRESENTE A LA SURFACE DES CELLULES SOUCHES HEMATOPOIETIQUES DE LA LIGNEE LYMPHOIDE ET DES CELLULES NK, ET SES APPLICATIONS.
<130> BLOcp263FR61S
<140>
   <141>
<150> 9914241
   <151> 2000-11-12
<160> 25
<170> PatentIn Ver. 2.1
<210> 1
   <211> 1501
   <212> ADN
   <213> Homo sapiens
<220>
   <221> CDS
   <222> (116)..(1168)
<400> 1
   cgcggccgcg tcgacctctc ctcgaccctg gacgtctacc ttccggaggc ccacatcttg 60
   cccactccgc gcgcggggct agcgcgggtt tcagcgacgg gagccctcaa gggac atg  118
   gca act aca gcg gcg ccg gcg ggc ggc gcc cga aat gga gct ggc ccg   166
   gaa tgg gga ggg ttc gaa gaa aac atc cag ggc gga ggc tca gct gtg   214
   att gac atg gag aac atg gat gat acc tca ggc tct agc ttc gag gat   262
   atg ggt gag ctg cat cag cgc ctg cgc gag gaa gaa gta gac gct gat   310
   gca gct gat gca gct gct gct gaa gag gag gat gga gag ttc ctg ggc   358
   atg aag ggc ttt aag gga cag ctg agc cgg cag gtg gca gat cag atg      406
   tgg cag gct ggg aaa aga caa gcc tcc agg gcc ttc agc ttg tac gcc      454
   aac atc gac atc ctc aga ccc tac ttt gat gtg gag cct gct cag gtg      502
   cga agc agg ctc ctg gag tcc atg atc cct atc aag atg gtc aac ttc      550
   ccc cag aaa att gca ggt gaa ctc tat gga cct ctc atg ctg gtc ctc      598
   act ctg gtt gct atc cta ctc cat ggg atg aag acg tct gac act att      646
   atc cgg gag ggc acc ctg atg ggc aca gcc att ggc acc tgc ttc ggc      694
   tac tgg ctg gga gtc tca tcc ttc att tac ttc ctt gcc tac ctg tgc      742
   aac gcc cag atc acc atg ctg cag atg ttg gca ctg ctg ggc tat ggc      790
   ctc ttt ggg cat tgc att gtc ctg ttc atc acc tat aat atc cac ctc      838
   cac gcc ctc ttc tac ctc ttc tgg ctg ttg gtg ggt gga ctg tcc aca      886
   ctg cgc atg gta gca gtg ttg gtg tct cgg acc gtg ggc ccc aca cag      934
   cgg ctg ctc ctc tgt ggc acc ctg gct gcc cta cac atg ctc ttc ctg      982
   ctc tat ctg cat ttt gcc tac cac aaa gtg gta gag ggg atc ctg gac      1030
   aca ctg gag ggc ccc aac atc ccg ccc atc cag agg gtc ccc aga gac    1078
   atc cct gcc atg ctc cct gct gct cgg ctt ccc acc acc gtc ctc aac    1126
   gcc aca gcc aaa gct gtt gcg gtg acc ctg cag tca cac tga            1168
   ccccacctga aattcttggc cagtcctctt tcccgcagct gcagagagga ggaagactat 1228
   taaaggacag tcctgatgac atgtttcgta gatggggttt gcagctgcca ctgagctgta 1288
   gctgcgtaag tacctccttg atgcctgtgg gcacttctga aaggcacaag gccaagaact 1348
   cctggccagg actgcaaggc tctgcagcca atgcagaaaa tgggtcaggt cctttgagaa 1408
   cccctcccca cctacccctt ccttcctctt tatctctccc acattgtctt ggtaaatata 1468
   gacttggtaa ttaaaatgtt gattgaagtc tgg                              1501
<210> 2
   <211> 350
   <212> PRT
   <213> Homo sapiens
<400> 2
<210> 3
   <211> 507
   <212> ADN
   <213> Mus musculus
<220>
   <221> CDS
   <222> (1)..(507)
<400> 3
   aac atc cag ggc ggg ggt tcg gct gtg att gat atg gag aac atg gac 48
   Asn Ile Gln Gly Gly Gly Ser Ala Val Ile Asp Met Glu Asn Met Asp
   1 5 10 15
   gat acc tca ggc tcc agc ttc gag gac atg ggt gag ctg cac cag cgc 96
   Asp Thr Ser Gly Ser Ser Phe Glu Asp Met Gly Glu Leu His Gln Arg
   20 25 30
   ctg cgg gag gaa gaa gta gat gct gat gca gct gct gca gaa gaa gag 144
   Leu Arg Glu Glu Glu Val Asp Ala Asp Ala Ala Ala Ala Glu Glu Glu
   35 40 45
   gat ggg gag ttt ctt ggc atg aaa ggc ttt aaa gga caa ctg agc cgg 192
   Asp Gly Glu Phe Leu Gly Met Lys Gly Phe Lys Gly Gln Leu Ser Arg
   50 55 60
   cag gta gca gat cag atg tgg cag gca ggg aag aga cag gct tcc agg 240
   Gln Val Ala Asp Gln Met Trp Gln Ala Gly Lys Arg Gln Ala Ser Arg
   65 70 75 80
   gcc ttc agc ttg tat gcc aac att gac atc ctc aga ccc tac ttt gat 288
   Ala Phe Ser Leu Tyr Ala Asn Ile Asp Ile Leu Arg Pro Tyr Phe Asp
   85 90 95
   gtg gag cct gcc cag gtc cga agc agg ctc ctg gag tcc atg atc cct 336
   Val Glu Pro Ala Gln Val Arg Ser Arg Leu Leu Glu Ser Met Ile Pro
   100 105 110
   atc aag atg gtc aac ttc ccc cag aaa gtc gcg ggc gag ctc tac gga 384
   Ile Lys Met Val Asn Phe Pro Gln Lys Val Ala Gly Glu Leu Tyr Gly
   115 120 125
   ccg ctc atg ctg gtc ttc aca ctg gtg gcc atc ctc ctg cat gga atg 432
   Pro Leu Met Leu Val Phe Thr Leu Val Ala Ile Leu Leu His Gly Met
   130 135 140
   aag act tct gac acc att atc cgg gag ggc acc ctc atg ggc aca gcc 480
   Lys Thr Ser Asp Thr Ile Ile Arg Glu Gly Thr Leu Met Gly Thr Ala
   145 150 155 160
   ata ggc acc tgc ttt gga tac tgg ctg 507
   Ile Gly Thr Cys Phe Gly Tyr Trp Leu
   165
<210> 4
   <211> 169
   <212> PRT
   <213> Mus musculus
<400> 4
   Asn Ile Gln Gly Gly Gly Ser Ala Val Ile Asp Met Glu Asn Met Asp
   1               5                   10                  15
   Asp Thr Ser Gly Ser Ser Phe Glu Asp Met Gly Glu Leu His Gln Arg
               20                  25                  30
   Leu Arg Glu Glu Glu Val Asp Ala Asp Ala Ala Ala Ala Glu Glu Glu
           35                  40                  45
   Asp Gly Glu Phe Leu Gly Met Lys Gly Phe Lys Gly Gln Leu Ser Arg
       50                  55                  60
   Gln Val Ala Asp Gln Met Trp Gln Ala Gly Lys Arg Gln Ala Ser Arg
   65                  70                  75                  80
   Ala Phe Ser Leu Tyr Ala Asn Ile Asp Ile Leu Arg Pro Tyr Phe Asp
                   85                  90                  95
   Val Glu Pro Ala Gln Val Arg Ser Arg Leu Leu Glu Ser Met Ile Pro
               100                 105                 110
   Ile Lys Met Val Asn Phe Pro Gln Lys Val Ala Gly Glu Leu Tyr Gly
           115                 120                 125
   Pro Leu Met Leu Val Phe Thr Leu Val Ala Ile Leu Leu His Gly Met
       130                 135                 140
   Lys Thr Ser Asp Thr Ile Ile Arg Glu Gly Thr Leu Met Gly Thr Ala
   145                 150                 155                 160
   Ile Gly Thr Cys Phe Gly Tyr Trp Leu
                   165
<210> 5
   <211> 471
   <212> ADN
   <213> Sus sp.
<220>
   <221> CDS
   <222> (1)..(471)
<220>
   <221> sig_peptide
   <222> (1)..(60)
<220>
   <221> misc_feature
   <222> (61)..(456)
<400> 5
   atg gca act aca gcg gcg ccg gcg ggc ggc gcc cga aat gga gct ggc    48
   Met Ala Thr Thr Ala Ala Pro Ala Gly Gly Ala Arg Asn Gly Ala Gly
     1               5                  10                  15
   ccg gaa tgg gga ggg ttc gaa gaa aac atc cag ggc gga ggc tca gct    96
   Pro Glu Trp Gly Gly Phe Glu Glu Asn Ile Gln Gly Gly Gly Ser Ala
                20                  25                  30
   gtg att gac atg gag aac atg gat gat acc tca ggc tct agc ttc gag   144
   Val Ile Asp Met Glu Asn Met Asp Asp Thr Ser Gly Ser Ser Phe Glu
            35                  40                  45
   gat atg ggt gag ctg cat cag cgc ctg cgc gag gaa gaa gta gac gct   192
   Asp Met Gly Glu Leu His Gln Arg Leu Arg Glu Glu Glu Val Asp Ala
        50                  55                  60
   gat gca gct gat gca gct gct gct gaa gag gag gat gga gag ttc ctg   240
   Asp Ala Ala Asp Ala Ala Ala Ala Glu Glu Glu Asp Gly Glu Phe Leu
    65                  70                  75                  80
   ggc atg aag ggc ttt aag gga cag ctg agc cgg cag gtg gca gat cag   288
   Gly Met Lys Gly Phe Lys Gly Gln Leu Ser Arg Gln Val Ala Asp Gln
                    85                  90                  95
   atg tgg cag gct ggg aaa aga caa gcc tcc agg gcc ttc agc ttg tac   336
   Met Trp Gln Ala Gly Lys Arg Gln Ala Ser Arg Ala Phe Ser Leu Tyr
               100                 105                 110
   gcc aac atc gac atc ctc aga ccc tac ttt gat gtg gag cct gct cag   384
   Ala Asn Ile Asp Ile Leu Arg Pro Tyr Phe Asp Val Glu Pro Ala Gln
           115                 120                 125
   gtg cga agc agg ctc ctg gag tcc atg atc cct atc aag atg gtc aac   432
   Val Arg Ser Arg Leu Leu Glu Ser Met Ile Pro Ile Lys Met Val Asn
       130                 135                 140
   ttc ccc cag aaa att gca ggt gaa ctc tat gga cct ctc               471
   Phe Pro Gln Lys Ile Ala Gly Glu Leu Tyr Gly Pro Leu
   145                 150                 155
<210> 6
   <211> 157
   <212> PRT
   <213> Sus sp.
<400> 6
   Met Ala Thr Thr Ala Ala Pro Ala Gly Gly Ala Arg Asn Gly Ala Gly
   1 5 10 15
   Pro Glu Trp Gly Gly Phe Glu Glu Asn Ile Gln Gly Gly Gly Ser Ala
   20 25 30
   Val Ile Asp Met Glu Asn Met Asp Asp Thr Ser Gly Ser Ser Phe Glu
   35 40 45
   Asp Met Gly Glu Leu His Gln Arg Leu Arg Glu Glu Glu Val Asp Ala
   50 55 60
   Asp Ala Ala Asp Ala Ala Ala Ala Glu Glu Glu Asp Gly Glu Phe Leu
   65 70 75 80
   Gly Met Lys Gly Phe Lys Gly Gln Leu Ser Arg Gln Val Ala Asp Gln
   85 90 95
   Met Trp Gln Ala Gly Lys Arg Gln Ala Ser Arg Ala Phe Ser Leu Tyr
   100 105 110
   Ala Asn Ile Asp Ile Leu Arg Pro Tyr Phe Asp Val Glu Pro Ala Gln
   115 120 125
   Val Arg Ser Arg Leu Leu Glu Ser Met Ile Pro Ile Lys Met Val Asn
   130 135 140
   Phe Pro Gln Lys Ile Ala Gly Glu Leu Tyr Gly Pro Leu
   145 150 155
<210> 7
   <211> 132
   <212> PRT
   <213> Homo sapiens
<400> 7
   Gly Phe Glu Glu Asn Ile Gln Gly Gly Gly Ser Ala Val Ile Asp Met
   1 5 10 15
   Glu Asn Met Asp Asp Thr Ser Gly Ser Ser Phe Glu Asp Met Gly Glu
   20 25 30
   Leu His Gln Arg Leu Arg Glu Glu Glu Val Asp Ala Asp Ala Ala Asp
   35 40 45
   Ala Ala Ala Ala Glu Glu Glu Asp Gly Glu Phe Leu Gly Met Lys Gly
   50 55 60
   Phe Lys Gly Gln Leu Ser Arg Gln Val Ala Asp Gln Met Trp Gln Ala
   65 70 75 80
   Gly Lys Arg Gln Ala Ser Arg Ala Phe Ser Leu Tyr Ala Asn Ile Asp
   85 90 95
   Ile Leu Arg Pro Tyr Phe Asp Val Glu Pro Ala Gln Val Arg Ser Arg
   100 105 110
   Leu Leu Glu Ser Met Ile Pro Ile Lys Met Val Asn Phe Pro Gln Lys
   115 120 125
   Ile Ala Gly Glu
   130
<210> 8
   <211> 143
   <212> PRT
   <213> Homo sapiens
<400> 8
   Leu Tyr Gly Pro Leu Met Leu Val Leu Thr Leu Val Ala Ile Leu Leu
   1 5 10 15
   His Gly Met Lys Thr Ser Asp Thr Ile Ile Arg Glu Gly Thr Leu Met
   20 25 30
   Gly Thr Ala Ile Gly Thr Cys Phe Gly Tyr Trp Leu Gly Val Ser Ser
   35 40 45
   Phe Ile Tyr Phe Leu Ala Tyr Leu Cys Asn Ala Gln Ile Thr Met Leu
   50 55 60
   Gln Met Leu Ala Leu Leu Gly Tyr Gly Leu Phe Gly His Cys Ile Val
   65 70 75 80
   Leu Phe Ile Thr Tyr Asn Ile His Leu His Ala Leu Phe Tyr Leu Phe
   85 90 95
   Trp Leu Leu Val Gly Gly Leu Ser Thr Leu Arg Met Val Ala Val Leu
   100 105 110
   Val Ser Arg Thr Val Gly Pro Thr Gln Arg Leu Leu Leu Cys Gly Thr
   115 120 125
   Leu Ala Ala Leu His Met Leu Phe Leu Leu Tyr Leu His Phe Ala
   130 135 140
<210> 9
   <211> 55
   <212> PRT
   <213> Homo sapiens
<400> 9
   Tyr His Lys Val Val Glu Gly Ile Leu Asp Thr Leu Glu Gly Pro Asn
    1               5                  10                  15
   Ile Pro Pro Ile Gln Arg Val Pro Arg Asp Ile Pro Ala Met Leu Pro
               20                  25                  30
   Ala Ala Arg Leu Pro Thr Thr Val Leu Asn Ala Thr Ala Lys Ala Val
           35                  40                  45
   Ala Val Thr Leu Gln Ser His
       50                  55
<210> 10
   <211> 1053
   <212> ADN
   <213> Homo sapiens
<400> 10
   atggcaacta cagcggcgcc ggcgggcggc gcccgaaatg gagctggccc ggaatgggga 60
   gggttcgaag aaaacatcca gggcggaggc tcagctgtga ttgacatgga gaacatggat 120
   gatacctcag gctctagctt cgaggatatg ggtgagctgc atcagcgcct gcgcgaggaa 180
   gaagtagacg ctgatgcagc tgatgcagct gctgctgaag aggaggatgg agagttcctg 240
   ggcatgaagg gctttaaggg acagctgagc cggcaggtgg cagatcagat gtggcaggct 300
   gggaaaagac aagcctccag ggccttcagc ttgtacgcca acatcgacat cctcagaccc 360
   tactttgatg tggagcctgc tcaggtgcga agcaggctcc tggagtccat gatccctatc 420
   aagatggtca acttccccca gaaaattgca ggtgaactct atggacctct catgctggtc 480
   ctcactctgg ttgctatcct actccatggg atgaagacgt ctgacactat tatccgggag 540
   ggcaccctga tgggcacagc cattggcacc tgcttcggct actggctggg agtctcatcc 600
   ttcatttact tccttgccta cctgtgcaac gcccagatca ccatgctgca gatgttggca 660
   ctgctgggct atggcctctt tgggcattgc attgtcctgt tcatcaccta taatatccac 720
   ctccacgccc tcttctacct cttctggctg ttggtgggtg gactgtccac actgcgcatg 780
   gtagcagtgt tggtgtctcg gaccgtgggc cccacacagc ggctgctcct ctgtggcacc 840
   ctggctgccc tacacatgct cttcctgctc tatctgcatt ttgcctacca caaagtggta 900
   gaggggatcc tggacacact ggagggcccc aacatcccgc ccatccagag ggtccccaga 960
   gacatccctg ccatgctccc tgctgctcgg cttcccacca ccgtcctcaa cgccacagcc 1020
   aaagctgttg cggtgaccct gcagtcacac tga                              1053
<210> 11
   <211> 456
   <212> ADN
   <213> Homo sapiens
<400> 11
   atggcaacta cagcggcgcc ggcgggcggc gcccgaaatg gagctggccc ggaatgggga 60
   gggttcgaag aaaacatcca gggcggaggc tcagctgtga ttgacatgga gaacatggat 120
   gatacctcag gctctagctt cgaggatatg ggtgagctgc atcagcgcct gcgcgaggaa 180
   gaagtagacg ctgatgcagc tgatgcagct gctgctgaag aggaggatgg agagttcctg 240
   ggcatgaagg gctttaaggg acagctgagc cggcaggtgg cagatcagat gtggcaggct 300
   gggaaaagac aagcctccag ggccttcagc ttgtacgcca acatcgacat cctcagaccc 360
   tactttgatg tggagcctgc tcaggtgcga agcaggctcc tggagtccat gatccctatc 420
   aagatggtca acttccccca gaaaattgca ggtgaa                           456
<210> 12
   <211> 429
   <212> ADN
   <213> Homo sapiens
<400> 12
   ctctatggac ctctcatgct ggtcctcact ctggttgcta tcctactcca tgggatgaag 60
   acgtctgaca ctattatccg ggagggcacc ctgatgggca cagccattgg cacctgcttc 120
   ggctactggc tgggagtctc atccttcatt tacttccttg cctacctgtg caacgcccag 180
   atcaccatgc tgcagatgtt ggcactgctg ggctatggcc tctttgggca ttgcattgtc 240
   ctgttcatca cctataatat ccacctccac gccctcttct acctcttctg gctgttggtg 300
   ggtggactgt ccacactgcg catggtagca gtgttggtgt ctcggaccgt gggccccaca 360
   cagcggctgc tcctctgtgg caccctggct gccctacaca tgctcttcct gctctatctg 420
   cattttgcc                                                         429
<210> 13
   <211> 168
   <212> ADN
   <213> Homo sapiens
<400> 13
   taccacaaag tggtagaggg gatcctggac acactggagg gccccaacat cccgcccatc 60
   cagagggtcc ccagagacat ccctgccatg ctccctgctg ctcggcttcc caccaccgtc 120
   ctcaacgcca cagccaaagc tgttgcggtg accctgcagt cacactga              168
<210> 14
   <211> 13
   <212> ADN
   <213> Homo sapiens
<400> 14
   tttttttttt tgg 13
<210> 15
   <211> 10
   <212> ADN
   <213> Homo sapiens
<400> 15
   gttgcgatcc 10
<210> 16
   <211> 20
   <212> ADN
   <213> Homo sapiens
<400> 16
   accccacctg aaattcttgg 20
<210> 17
   <211> 20
   <212> ADN
   <213> Homo sapiens
<400> 17
   gagataaaga ggaaggaagg 20
<210> 18
   <211> 20
   <212> ADN
   <213> Homo sapiens
<400> 18
   ctgtgggcac ttctgaaagg 20
<210> 19
   <211> 20
   <212> ADN
   <213> Homo sapiens
<400> 19
   tcgaagaaaa catccagggc 20
<210> 20
   <211> 21
   <212> ADN
   <213> Homo sapiens
<400> 20
   agaggtccat agagttccac c 21
<210> 21
   <211> 49
   <212> ADN
   <213> Homo sapiens
<400> 21
   agatcctaat acgactcact atagggagga gggacatggc caactaagc 49
<210> 22
   <211> 20
   <212> ADN
   <213> Homo sapiens
<400> 22
   aagaggaagg aaggggtagg 20
<210> 23
   <211> 387
   <212> ADN
   <213> Mus musculus
<220>
   <221> CDS
   <222> (1)..(387)
<400> 23
   ggg ttc gaa gaa aac atc cag ggc ggg ggt tcg gct gtg att gat atg   48
   Gly Phe Glu Glu Asn Ile Gln Gly Gly Gly Ser Ala Val Ile Asp Met
    1               5                  10                  15
   gag aac atg gac gat acc tca ggc tcc agc ttc gag gac atg ggt gag     96
   Glu Asn Met Asp Asp Thr Ser Gly Ser Ser Phe Glu Asp Met Gly Glu
               20                  25                  30
   ctg cac cag cgc ctg cgg gag gaa gaa gta gat gct gat gca gct gct    144
   Leu His Gln Arg Leu Arg Glu Glu Glu Val Asp Ala Asp Ala Ala Ala
           35                  40                  45
   gca gaa gaa gag gat ggg gag ttt ctt ggc atg aaa ggc ttt aaa gga    192
   Ala Glu Glu Glu Asp Gly Glu Phe Leu Gly Met Lys Gly Phe Lys Gly
       50                  55                  60
   caa ctg agc cgg cag gta gca gat cag atg tgg cag gca ggg aag aga    240
   Gln Leu Ser Arg Gln Val Ala Asp Gln Met Trp Gln Ala Gly Lys Arg
   65                  70                  75                  80
   cag gct tcc agg gcc ttc agc ttg tat gcc aac att gac atc ctc aga    288
   Gln Ala Ser Arg Ala Phe Ser Leu Tyr Ala Asn Ile Asp Ile Leu Arg
                   85                  90                  95
   ccc tac ttt gat gtg gag cct gcc cag gtc cga agc agg ctc ctg gag    336
   Pro Tyr Phe Asp Val Glu Pro Ala Gln Val Arg Ser Arg Leu Leu Glu
               100                 105                 110
   tcc atg atc cct atc aag atg gtc aac ttc ccc cag aaa gtc gcg ggc    384
   Ser Met Ile Pro Ile Lys Met Val Asn Phe Pro Gln Lys Val Ala Gly
           115                 120                 125
   gag                                                                387
   Glu
<210> 24
   <211> 129
   <212> PRT
   <213> Mus musculus
<400> 24
   Gly Phe Glu Glu Asn Ile Gln Gly Gly Gly Ser Ala Val Ile Asp Met
   1               5                   10                  15
   Glu Asn Met Asp Asp Thr Ser Gly Ser Ser Phe Glu Asp Met Gly Glu
               20                  25                  30
   Leu His Gln Arg Leu Arg Glu Glu Glu Val Asp Ala Asp Ala Ala Ala
           35                  40                  45
   Ala Glu Glu Glu Asp Gly Glu Phe Leu Gly Met Lys Gly Phe Lys Gly
       50                  55                  60
   Gln Leu Ser Arg Gln Val Ala Asp Gln Met Trp Gln Ala Gly Lys Arg
   65                  70                  75                  80
   Gln Ala Ser Arg Ala Phe Ser Leu Tyr Ala Asn Ile Asp Ile Leu Arg
                   85                  90                  95
   Pro Tyr Phe Asp Val Glu Pro Ala Gln Val Arg Ser Arg Leu Leu Glu
   100 105 110
   Ser Met Ile Pro Ile Lys Met Val Asn Phe Pro Gln Lys Val Ala Gly
   115 120 125
   Glu
<210> 25
   <211> 330
   <212> PRT
   <213> Homo sapiens
<400> 25
   Gly Phe Glu Glu Asn Ile Gln Gly Gly Gly Ser Ala Val Ile Asp Met
    1               5                  10                  15
   Glu Asn Met Asp Asp Thr Ser Gly Ser Ser Phe Glu Asp Met Gly Glu
               20                  25                  30
   Leu His Gln Arg Leu Arg Glu Glu Glu Val Asp Ala Asp Ala Ala Asp
           35                  40                  45
   Ala Ala Ala Ala Glu Glu Glu Asp Gly Glu Phe Leu Gly Met Lys Gly
       50                  55                  60
   Phe Lys Gly Gln Leu Ser Arg Gln Val Ala Asp Gln Met Trp Gln Ala
   65                  70                  75                  80
   Gly Lys Arg Gln Ala Ser Arg Ala Phe Ser Leu Tyr Ala Asn Ile Asp
                   85                  90                  95
   Ile Leu Arg Pro Tyr Phe Asp Val Glu Pro Ala Gln Val Arg Ser Arg
               100                 105                 110
   Leu Leu Glu Ser Met Ile Pro Ile Lys Met Val Asn Phe Pro Gln Lys
           115                 120                 125
   Ile Ala Gly Glu Leu Tyr Gly Pro Leu Met Leu Val Leu Thr Leu Val
       130                 135                 140
   Ala Ile Leu Leu His Gly Met Lys Thr Ser Asp Thr Ile Ile Arg Glu
   145                 150                 155                 160
   Gly Thr Leu Met Gly Thr Ala Ile Gly Thr Cys Phe Gly Tyr Trp Leu
                   165                 170                 175
   Gly Val Ser Ser Phe Ile Tyr Phe Leu Ala Tyr Leu Cys Asn Ala Gln
               180                 185                 190
   Ile Thr Met Leu Gln Met Leu Ala Leu Leu Gly Tyr Gly Leu Phe Gly
           195                 200                 205
   His Cys Ile Val Leu Phe Ile Thr Tyr Asn Ile His Leu His Ala Leu
       210                 215                 220
   Phe Tyr Leu Phe Trp Leu Leu Val Gly Gly Leu Ser Thr Leu Arg Met
   225                 230                 235                 240
   Val Ala Val Leu Val Ser Arg Thr Val Gly Pro Thr Gln Arg Leu Leu
                   245                 250                 255
   Leu Cys Gly Thr Leu Ala Ala Leu His Met Leu Phe Leu Leu Tyr Leu
               260                 265                 270
   His Phe Ala Tyr His Lys Val Val Glu Gly Ile Leu Asp Thr Leu Glu
           275                 280                 285
   Gly Pro Asn Ile Pro Pro Ile Gln Arg Val Pro Arg Asp Ile Pro Ala
       290                 295                 300
   Met Leu Pro Ala Ala Arg Leu Pro Thr Thr Val Leu Asn Ala Thr Ala
   305                 310                 315                 320
   Lys Ala Val Ala Val Thr Leu Gln Ser His
                   325                 330

## Description

La présente invention est relative à une protéine présente à la surface des cellules souches hématopoïétiques de la lignée lymphoïde (cellules pré-T, cellules pré-B et cellules pré- NK) et des cellules NK matures, à la séquence isolée d'ADNc correspondante ainsi qu'à leurs applications en tant que marqueur desdites cellules et pour la préparation d'anticorps dirigés contre ladite protéine ; la présente invention est également relative aux applications desdits anticorps dans la sélection de cellules exprimant à leur surface ladite protéine.

De tels anticorps sont notamment utiles dans la déplétion de la moelle osseuse ou du sang de cordon ombilical en cellules NK, avant une greffe de moelle osseuse, en vue de réduire le rejet de cette dernière et la GVHD.

Les cellules NK représentent 10 à 15 % des cellules mononucléées du sang périphérique. On admet qu'elles constituent une troisième lignée lymphocytaire à côté des cellules B et T ; elles ont été définies comme de gros lymphocytes à grains (LGL) qui n'expriment à leur surface ni le complexe moléculaire CD3, ni les chaînes α, β, γ du récepteur T, portent les marqueurs CD16 et CD56 chez l'homme, NK1.1/NK2.1 chez la souris et exercent une action cytolytique qui ne requiert pas l'expression d'antigènes de classe 1 ou II du CMH à la surface des cellules cibles.

On ne connaît pas d'antigène de surface exprimé uniquement par les progéniteurs de la lignée lymphoïde (cellules pré-T, pré-B et pré-NK) et les cellules NK matures. Deux marqueurs sont utilisés pour distinguer opérationnellement les cellules NK des autres populations lymphocytaires. Il s'agit de l'antigène CD56 et du récepteur de basse affinité pour le fragment Fc des immunoglobulines ou CD16.

Bien que la plupart des cellules NK puissent être considérées comme CD3-, CD16+, CD56+, il existe une grande hétérogénéité phénotypique et fonctionnelle dans cette population de cellules ; la plupart des lymphocytes CD56+ du sang périphérique expriment aussi la molécule CD16 ; environ 10% des cellules NK du sang périphérique sont CD16-, tout en exprimant à haute densité le CD56. Il a été suggéré que les lymphocytes CD16- CD56^{fort} pourraient être les précurseurs des lymphocytes NK CD16+ CD56^{faible}.

Toutefois, dans la mesure où certaines cellules T expriment à la fois la molécule CD16 et la molécule CD56, ces deux molécules ne sont pas spécifiques de la population des cellules NK du sang périphérique.

Les cellules NK présentent une activité cytolytique naturelle qui s'exerce directement, sans nécessiter d'immunisation préalable et ne requiert pas la présence sur la cellule cible d'antigènes du complexe majeur d'histocompatibilité et une cytotoxicité cellulaire dépendante des anticorps (ADCC). L'ADCC est initiée par la liaison de l'anticorps fixé sur la cellule cible avec le récepteur Fc (CD16) de la cellule effectrice. Les cellules NK jouent donc un rôle important dans la défense de l'hôte contre les infections virales et dans la surveillance immune vis-à-vis des cellules tumorales.

Les cellules NK peuvent induire une forme primitive d'alloreconnaissance qui contribue au rejet de la greffe lors d'une transplantation allogénique ainsi qu'à une maladie du greffon contre l'hôte (GVHD ou « *graft versus host disease* »). Pour ces raisons une identification fiable des cellules NK dans la population de cellules mononucléées ainsi que de l'ensemble des cellules précurseurs de la lignée lymphoïde est importante.

Un certain nombre de documents proposent des méthodes de déplétion de la moelle osseuse en cellules NK ; dans ces différentes méthodes, les cellules NK sont sélectionnées au moyen des marqueurs tels que CD56, CD16, PEN5, NKB1 :
* la Demande Internationale PCT WO 95/20604 décrit un antigène présent sur les cellules NK et les cellules T. Cet antigène a été dénommé NKB1. Un anticorps monoclonal dirigé contre cet antigène est également décrit (anticorps DX9). Ce marqueur NKB 1 présente les propriétés suivantes :
   - il se lie spécifiquement à l'anticorps DX9,
   - il présente un poids moléculaire de l'ordre de 50 kDa,
   - il s'agit d'une protéine glycosylée ; le poids moléculaire de la protéine glycosylée est d'environ 70 kDa,
   - la forme naturelle de la protéine contient des résidus d'acide sialique et est notamment phosphorylée,
   - les marqueurs NKB1 sont exprimés par un sous-ensemble de cellules NK et par un sous-ensemble de cellules T.
* la Demande Internationale PCT WO 95/06247 décrit un antigène spécifique des cellules NK sous la forme d'une paire de glycoprotéines, désignées PEN5α et PEN5β, ayant respectivement un poids moléculaire apparent de 120-150 et 210-245 kDa.

Les épitopes uniques de la paire de glycoprotéines PEN5α/PEN5β sont exprimés de préférence par une sous-population de cellules NK du sang périphérique de phénotype CD16+ CD56^{dim} ; ils ne sont pas présents sur les lymphocytes T CD3+ ou les lymphocytes B CD20+.

Ces méthodes aboutissent à la déplétion des cellules NK matures CD56+ CD16+ ou prématures PEN5 NKB1, mais ne déplètent pas les progéniteurs, lesquels, après la greffe peuvent se différencier en cellules matures qui vont provoquer le rejet des greffes.

D'autres documents confirment cette absence de marqueurs spécifiques ; par exemple, dans l'article au nom de E. Dominguez et al. (Immunol., 1998, 94, 109-114), il est précisé qu'aucun marqueur unique des cellules NK n'a encore été identifié et qu'on les distingue, de manière habituelle par le fait qu'elles expriment CD56.

Il ressort de l'ensemble de ces documents qu'il n'existe pas de marqueurs spécifiques des cellules NK d'une part, ni de l'ensemble des progéniteurs de la lignée lymphoïde d'autre part.

La Demanderesse s'est donc donné pour but de pourvoir à un marqueur spécifique des cellules NK matures ainsi que de l'ensemble des cellules non différenciées de la lignée lymphoïde, afin de permettre la déplétion notamment de la moelle osseuse en cellules précurseurs de la lignée lymphoïde (pré NK, pré T et pré B) et en cellules NK matures, en particulier avant une greffe de moelle osseuse, en vue de réduire le rejet de cette dernière et la GVHD.

En effet, de manière surprenante, les Inventeurs ont maintenant trouvé qu'il existe un marqueur spécifique à la fois de l'ensemble des lignées progénitrices lymphoïdes (pré-T, pré-B et pré-NK) et des cellules NK matures.

La présente invention a pour objet une protéine isolée, **caractérisée :**
- **en ce qu**'elle est présente à la surface de l'ensemble des cellules progénitrices lymphoïdes et des cellules NK matures,
- en ce qu'elle présente une structure (a) comprenant un domaine extracellulaire situé entre les positions 21 et 152, cinq domaines transmembranaires situés entre les positions 153 et 295 et un domaine cytoplasmique situé entre les positions 296 et 350, en référence à la séquence SEQ ID NO:2,
- en ce qu'elle présente un poids moléculaire apparent d'environ 36-38 kDa, et
- en ce que son précurseur, qui comprend en N-terminal de la structure (a) une séquence signal de 20 acides aminés, est sélectionné dans le groupe constitué par la protéine de SEQ ID NO:2, les protéines présentant une séquence en acides aminés ayant au moins 70 % d'identité ou au moins 85% de similarité et de préférence au moins 95 % d'identité ou au moins 99 % de similarité avec la séquence SEQ ID NO:2.

On entend par précurseur, la protéine incluant la séquence signal.

« L'identité » d'une séquence par rapport à une séquence de référence s'apprécie en fonction du pourcentage de résidus d'acides aminés qui sont identiques, lorsque les deux séquences sont alignées de manière à obtenir le maximum de correspondance entre elles.

Une protéine ayant une séquence en acides aminés ayant au moins X % d'identité avec une séquence de référence est définie, dans la présente invention comme une protéine dont la séquence peut inclure jusqu'à 100-X altérations pour 100 acides aminés de la séquence de référence. Au sens de la présente invention, le terme altérations inclut les délétions, les substitutions ou les insertions consécutives ou dispersées d'acides aminés dans la séquence de référence.

« La similarité » d'une séquence par rapport à une séquence de référence s'apprécie en fonction du pourcentage de résidus d'acides aminés qui sont identiques ou qui diffèrent par des substitutions conservatives, lorsque les deux séquences sont alignées de manière à obtenir le maximum de correspondance entre elles. Au sens de la présente invention, on entend par substitution conservative, la substitution d'un acide aminé par un autre qui présente des propriétés chimiques similaires (taille, charge ou polarité), qui généralement ne modifie pas les propriétés fonctionnelles de la protéine.

Une protéine ayant une séquence en acides aminés ayant au moins X % de similarité avec une séquence de référence est définie, dans la présente invention comme une protéine dont la séquence peut inclure jusqu'à 1 00-X altérations non-conservatives pour 100 acides aminés de la séquence de référence. Au sens de la présente invention, le terme altérations non-conservatives inclut les délétions, les substitutions non-conservatives ou les insertions consécutives ou dispersées d'acides aminés dans la séquence de référence.

Un certain nombre de documents décrivent des protéines présentant une certaine homologie avec les protéines selon l'invention :
- la Demande PCT WO 98/39448 (Human Genome Sciences Inc) décrit le gène 177, clone HE9CM64 qui code potentiellement pour une protéine sécrétée (séquences nucléiques : SEQ ID NO:187 et 304, séquences peptidiques : SEQ ID NO:489 et 606) ; toutefois, seul l'ARNm a été détecté dans le cerveau et dans une moindre mesure dans le rein, le placenta, le muscle lisse, le coeur et le poumon. La protéine potentiellement codée par ledit ARNm n'a jamais été isolée et aucune fonction réelle n'est associée à cette protéine, qui n'est pas clairement définie, au niveau de ses extrémités N- et C-terminales qui peuvent varier. Ces séquences sont néanmoins exclues de la présente Demande par un disclaimer.
- les Demandes Internationales PCT WO 98/25959 (Chiron Corp.); PCT WO 99/31236 (Genset), ainsi que les informations relatives aux séquences accessibles dans la base DATABASE SWALL, sous le n° Q9Y3U9, ou les informations relatives aux séquences accessibles dans la base DATABASE EMEST, sous le n° AW106427 décrivent des fragments de séquences présentant une homologie avec un fragment de la séquence SEQ ID NO:2 ; toutefois, dans la demande en instance, l'homologie est clairement définie par rapport à la séquence SEQ ID NO:2 complète ; en outre, aucun de ces documents ne divulgue des protéines qui sont des marqueurs de surface des progéniteurs précoces de la lignée lymphoïde et des cellules NK matures.

En effet, la protéine selon l'invention présente les propriétés suivantes :
- elle est présente à la surface des cellules de la lignée de différenciation des cellules NK, des progéniteurs très précoces aux cellules matures cytotoxiques et des autres progéniteurs précoces de la lignée lymphoïde (pré-B et pré-T) ; ainsi, elle est exprimée à la surface de l'ensemble des cellules progénitrices lymphoïdes et des cellules NK matures.
- elle présente un poids moléculaire apparent d'environ 36-38 kDa, mesuré par électrophorèse SDS-PAGE à 12% dans des conditions réductrices,
- elle présente une structure (a) comprenant un domaine extracellulaire situé entre les positions 21 et 152, cinq domaines transmembranaires situés entre les positions 153 et 295 et un domaine cytoplasmique situé entre les positions 296 et 350, en référence à la séquence SEQ ID NO : 2, et
- son précurseur, qui comprend en N-terminal de la structure en (a) une séquence signal de 20 acides aminés, est sélectionné dans le groupe constitué par la séquence SEQ ID NO : 2 et les variants de cette séquence présentant au moins 70 % d'identité ou au moins 85 % de similarité et de préférence au moins 95 % d'identité ou au moins 99 % de similarité sur la longueur total de ladite séquence SEQ ID NO: 2, à l'exclusion des séquences SEQ ID NO :489 et 606 décrites dans la Demande Internationale PCT WO 98/39448.

Elle est de préférence sélectionnée dans le groupe constitué par la protéine présentant la formule SEQ ID NO:25 (protéine humaine, qui comprend 330 acides aminés) et les protéines d'autres mammifères dont le domaine extracellulaire est constitué par les fragments de séquence SEQ ID NO:6, 7 ou 24.

Ladite protéine ou molécule de surface, dénommée antigène KLIP-1 est donc spécifiquement présente sur la lignée de différenciation des cellules NK, des progéniteurs très précoces aux cellules matures cytotoxiques, ainsi que sur les autres progéniteurs précoces de la lignée lymphoïde (pré-B et pré-T).

En référence à la SEQ ID NO:2 humaine, les acides aminés 1-20 correspondent à la séquence signal ; les acides aminés 21-152 (SEQ ID NO:7 humaine ; SEQ ID NO:24 murine ; SEQ ID NO:6 porcine) correspondent au domaine extracellulaire ; les acides aminés 153-295 (SEQ ID NO:8) correspondent aux cinq domaines transmembranaires (143 acides aminés) et les acides aminés 296-350 (SEQ ID NO:9) correspondent au domaine cytoplasmique (54 acides aminés).

Les cellules suivantes sont KLIP-1+ :
- des progéniteurs hématopoïétiques précoces CD34+ CD38- de la moelle osseuse, de cordon ombilical et de foie foetal
- des progéniteurs différenciés CD34+ CD38+
- des progéniteurs pré-T (thymus néonatal) CD4+ CD8+
- des progéniteurs pré-B CD 19+, CD10-, CD20-
- des cellules NK matures CD56+ CD16+ CD19- CD3- CD33-.

Le fait que cet antigène KLIP-1 soit présent aussi bien sur les progéniteurs les plus précoces que sur les progéniteurs différenciés et les cellules NK matures permet d'obtenir une déplétion de la moelle osseuse en cellules NK en présence d'anticorps anti-KLIP-1, ce qui constitue une meilleure prévention du rejet des greffes et un traitement potentiel des maladies auto-immunes.

L'antigène KLIP-1 tel que décrit ci-dessus peut être considéré comme un marqueur spécifique des cellules NK (précoces et matures) et notamment un marqueur des cellules matures, dans la mesure où dans le sang périphérique, aucune autre cellule de la lignée lymphoïde (cellules B et cellules T matures) ne présente un tel antigène.

La présente invention a également pour objet des fragments de ladite protéine, **caractérisés en ce qu**'ils sont sélectionnés dans le groupe constitué par des fragments comprenant entre 7 acides aminés et 132 acides aminés consécutifs du domaine extracellulaire de ladite protéine, les fragments comprenant entre 7 acides aminés et 143 acides aminés consécutifs inclus dans les domaines transmembranaires de ladite protéine et les fragments comprenant entre 7 acides aminés et 54 acides aminés consécutifs inclus dans le domaine cytoplasmique de ladite protéine et le fragment de séquence SEQ ID NO:4 ; parmi lesdits fragments, on peut citer les séquences SEQ ID NO:6, 7, 8, 9 et 24.

De préférence, ledit fragment est sélectionné dans le groupe constitué par :
a) un peptide de 7 à 132 acides aminés consécutifs du domaine extracellulaire tel que défini ci-dessus,
b) un peptide de 7 à 143 acides aminés consécutifs des domaines transmembranaires tels que définis ci-dessus,
c) un peptide de 7 à 54 acides aminés consécutifs du domaine cytoplasmique tel que défini ci-dessus, et
d) un peptide de séquence SEQ ID NO : 4 ou 6.

La présente invention a également pour objet l'ADNc isolé et purifié codant pour ladite protéine ou ses fragments, tels que définis ci-dessus, d'origine humaine ou animale ainsi que les séquences d'acides nucléiques complémentaires.

Parmi ces séquences, on peut citer :
- l'ADNc codant pour l'antigène KLIP-1 humain, qui présente la séquence SEQ ID NO:1 et comprend 1501 pb ; il est obtenu à partir du gène klip-1, localisé sur le chromosome 6, dans la région 6p21.2-6p21.1 ;
- la séquence codant pour la protéine KLIP-1 humaine, qui est comprise entre la base 116 et la base 1165 de la séquence SEQ ID NO :1 (SEQ ID NO :10) ;
- la séquence codant pour le domaine extracellulaire de la protéine KLIP-1 humaine (SEQ ID NO:11), murine (SEQ ID NO:3 et SEQ ID NO:23) ou porcine (SEQ ID NO:5) ;
- la séquence codant pour les domaines transmembranaires de la protéine KLIP-1 humaine (SEQ ID NO:12) ; les positions des 5 hélices transmembranaires sont les suivantes : 153-169, 181-209, 218-237, 243-261, 275-295 ; et
- la séquence codant pour le domaine cytoplasmique de la protéine KLIP-1 humaine (SEQ ID NO:13).

La présente invention a également pour objet des fragments d'acide nucléique, **caractérisés en ce qu**'ils comprennent au moins 8 pb des différentes séquences d'ADNc telles que définies ci-dessus. De tels fragments peuvent notamment être utilisés comme sondes ou comme amorces d'amplification ou de transcription inverse, par exemple les séquences SEQ ID NO:16-20.

La présente invention a également pour objet des anticorps, caractérisés en ce qu'ils reconnaissent seulement une ou plusieurs protéines telles que définies ci-dessus et notamment l'antigène de surface KLIP-1 selon l'invention, spécifique de la lignée de différenciation des cellules NK, des progéniteurs très précoces aux cellules matures cytotoxiques et des autres progéniteurs précoces de la lignée lymphoïde (pré-B et pré-T) ou un fragment de celui-ci.

Selon un mode de réalisation avantageux desdits anticorps, ils sont dirigés contre un peptide du domaine extracellulaire de la protéine KLIP-1, tel que défini ci-dessus.

Conformément à l'invention, lesdits anticorps sont soient des anticorps polyclonaux, soient des anticorps monoclonaux.

De plus, les anticorps anti-KLIP-1 permettent également d'obtenir une déplétion en cellules progénitrices lymphoïdes (pré-B et pré-T).

Dans la transplantation de moelle osseuse, les anticorps, fragments et dérivés selon l'invention peuvent être utilisés pour dépléter la moelle osseuse en cellules KLIP-1+ *ex vivo*, avant la transplantation de la moelle du donneur dans la moelle du receveur.

A l'heure actuelle, il n'existe pas de marqueur des cellules précoces NK : KLIP-1 constitue ainsi le premier marqueur présent à la fois sur toute la lignée de la différenciation des cellules NK et sur les autres lignées lymphoïdes précoces.

Ainsi, la présente invention a également pour objet l'utilisation d'une protéine telle que définie ci-dessus, comme marqueur de surface des cellules NK matures et/ou des progéniteurs de la lignée lymphoïde.

La présente invention a également pour objet une méthode d'élimination sélective des cellules exprimant un marqueur tel que défini ci-dessus (KLIP-1+) à partir d'un échantillon biologique comprenant une population hétérogène de cellules, laquelle méthode comprend :
- la mise en contact dudit échantillon biologique avec un anticorps tel que défini ci-dessus et
- l'élimination des cellules qui se lient audit anticorps.

Selon un mode de mise en oeuvre avantageux dudit procédé, ledit anticorps est fixé sur un support solide tel que des billes magnétiques ou d'autres supports immunoadsorbants (Sepharose CNBr, par exemple).

Conformément audit mode de mise en oeuvre, pour dépléter l'échantillon biologique et notamment la moelle osseuse, le sang de cordon ou le foie foetal, en cellules comportant l'antigène KLIP-1 à leur surface, ces cellules sont fixées sur des billes immunomagnétiques, recouvertes d'anticorps anti-KLIP-1, de préférence des anticorps dirigés contre le domaine extracellulaire de la protéine KLIP-1.

Avantageusement, ladite méthode peut être mise en oeuvre pour enrichir un échantillon biologique en cellules NK humaines progénitrices ou matures à partir de mélanges de cellules humaines provenant de foie foetal, de sang de cordon ombilical, de moelle osseuse foetale, néonatale ou adulte ou de sang périphérique.

La présente invention a en outre pour objet l'utilisation des cellules de la moelle osseuse, du sang de cordon ombilical, de foie foetal ou de tout autre tissu hématopoïétique foetal ou adulte déplétées en cellules NK et en progéniteurs lymphoïdes, obtenues à l'aide du procédé tel que défini ci-dessus pour la préparation d'un produit apte à être transplanté.

La présente invention a également pour objet une méthode de détection et/ou de quantification et/ou d'isolement des cellules NK et/ou des progéniteurs des cellules lymphoïdes (pré-B, pré-T et pré-NK) dans une population hétérogène de cellules, laquelle méthode est **caractérisée en ce qu**'elle comprend :
- la mise en contact d'un échantillon biologique comprenant une population hétérogène de cellules avec un anticorps tel que défini ci-dessus, et
- la détection de la formation du complexe cellules-anticorps, notamment par une méthode immunologique standard.

Selon un mode de mise en oeuvre avantageux de ladite méthode, lorsque ledit échantillon est constitué de moelle osseuse, de sang de cordon ombilical, de foie foetal ou de tout autre tissu hématopoïétique (foetal ou adulte), elle peut comprendre en outre la mise en contact des cellules ayant formé un complexe avec ledit anticorps avec un anticorps spécifique des cellules pré-B (anticorps anti-CD19 ou anticorps anti-CD10) et/ou des cellules pré-T (anticorps anti-CD3, anticorps anti-CD4 ou anticorps anti-CD8).

La présente invention a également pour objet l'utilisation des anticorps tels que définis ci-dessus, dans le traitement des maladies auto-immunes.

La présente invention a également pour objet les anticorps tels que définis ci-dessus pour une utilisation comme réactif de détection et/ou de quantification et/ou d'isolement des cellules NK et/ou des progéniteurs de la lignée lymphoïde.

La présente invention a également pour objet une méthode de diagnostic des hémopathies malignes et bénignes, **caractérisée en ce qu**'elle comprend la détection dans un échantillon biologique d'une séquence nucléique telle que définie ci-dessus.

En effet, la détermination de la modulation de l'expression du gène KLIP-1 et/ou des mutations de la séquence dudit gène permettent de diagnostiquer une hémopathie.

La présente invention a de plus pour objet une trousse de détection et/ou de quantification et/ou d'isolement des cellules NK et/ou des progéniteurs des cellules lymphoïdes (pré-B, pré-T et pré-NK), **caractérisée en ce qu**'elle comprend outre des quantités utiles de tampons appropriés pour la mise en oeuvre de ladite détection, des doses appropriées d'un anticorps tel que défini ci-dessus, éventuellement conjugué à un support solide immunoadsorbant et des doses appropriées d'un réactif de révélation du complexe cellules-anticorps éventuellement formé.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :
- la figure 1 représente une analyse différentielle de transcrits sur gel (*RNA differential display* (RDD)) ; à cette figure, les pistes 1 correspondent au sac vitellin, les pistes 2-5 correspondent au foie foetal et la piste 6 correspond à un contrôle ;
- la figure 2 représente les séquences nucléotidique et protéique humaines de KLIP-1 ; la figure 2' représente un modèle pour la partie transmembranaire ;
- la figure 3 représente la localisation chromosomique du gène KLIP-1 ;
- la figure 4 illustre l'induction d'expression d'une protéine de fusion comprenant le domaine extracellulaire de la protéine KLIP-1 humaine (12 kDa) ;
- la figure 5 illustre un Western blot sur les populations KLIP-1+ et KLIP-1- ;
- la figure 6 illustre une immunoprécipitation de la protéine transcrite et traduite *in vitro* ;
- la figure 7 représente la répartition des cellules KLIP-1+ dans du sang périphérique, contenant uniquement des cellules sanguines matures ;
- la figure 8 représente la répartition des cellules KLIP-1+ dans du sang de cordon ombilical, contenant en majorité des cellules sanguines matures mais aussi une faible fraction des cellules immatures ;
- la figure 9 représente la répartition des cellules KLIP-1+ dans de la moelle osseuse, un site d'hématopoïèse où s'effectue la différenciation des cellules sanguines ;
- la figure 10 représente la répartition des cellules KLIP-1+ dans le foie embryonnaire (6 semaines) ;
- la figure 11 représente la répartition des cellules KLIP-1+ dans le thymus néo-natal ;
- la figure 12 illustre les propriétés de cytolyse des cellules NK et de la population NK purifiée par KLIP-1+ (cellules cibles) ;
- la figure 13 illustre la répartition des cellules KLIP-1+ dans le sang périphérique du porc ; et
- la figure 14 illustre la répartition des cellules KLIP-1+ dans les splénocytes de souris considérées comme des cellules NK (formation de complexes avec des anticorps NK 1.1 (Pharmingen)).

Il doit être bien entendu, toutefois, que ces exemples sont donnés uniquement à titre d'illustration de l'objet de l'invention, dont ils ne constituent en aucune manière une limitation.

### EXEMPTE :

### - Procédés cellulaires et tissulaires

Les tissus embryonnaires humains ont été obtenus à partir d'interruptions volontaires de grossesses, en accord avec la législation française. Le projet a été approuvé par le comité d'éthique local, et le consentement informé et préalable a été obtenu de toutes les mères. Le stade de développement (de gestation) de chaque embryon a été déterminé par des critères anatomiques. Le sac vitellin, l'aorte dorsale avec la splanchnopleure (région AGM) et le foie ont été disséqués sous une loupe binoculaire et immédiatement préparés pour l'extraction de l'ARN ou ont été congelés dans l'azote liquide et stockés à -80°C. En raison des différences de stade de développement des embryons, les tissus disséqués ont été traités séparément et non mélangés.

Le sang du cordon ombilical a été collecté à partir de nouveaux nés en bonne santé (sains) et à terme, immédiatement après l'accouchement avec l'accord de la mère.

La moelle osseuse et le sang périphérique ont été collectés à partir de donneurs adultes normaux, en accord avec les lignes directrices institutionnelles. Les cellules mononucléées ont été obtenues par centrifugation en gradient Ficoll-Hypaque dans des conditions standards. Les thymocytes postnatals normaux ont été isolés par élution à partir de morceaux de thymus prélevés lors d'opérations correctrices du coeur. Pour l'analyse cytofluoromérique, les cellules mononucléées ont été utilisées fraîches ou congelées.

### - Analyse différentielle de transcrits sur gel (RDD ou RNA differential display), clonage et séquençage

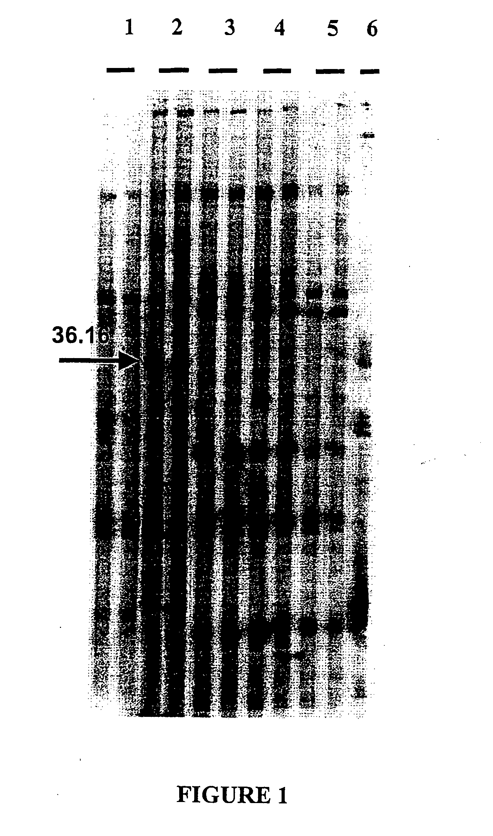

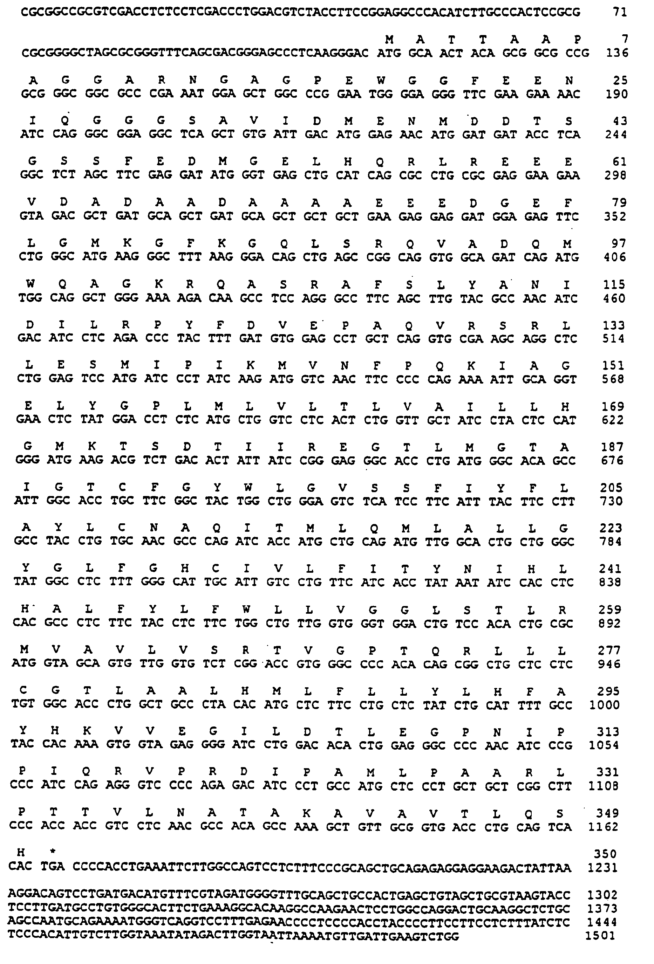

L'ARNm total a été extrait des tissus examinés (frais ou congelés) en utilisant le réactif RNA NOW (Ozyme, France), en accord avec les recommandations du fabricant et traité avec de la DNAase I sans RNAses (Boehringer Mannheim, Meylan, France) pendant 1 heure à 37 °C puis précipité à l'éthanol. La qualité de l'ARN a été contrôlée par électrophorèse en gel dénaturant d'agarose 1,5 %. Les ADNc ont été préparés à partir de 5 µg d'ARN total, en présence de 5'T₁₁XY(X=A, G ou C; Y=A, T, G ou C) (SEQ ID NO:14) comme amorce 3' d'ancrage et de transcriptase inverse M-MLV (GIBCO-BRL, Life Technologies, Cergy, France), à 42 °C pendant 1 heure. Les amplifications par PCR inverse ont été effectuées avec la même amorce 3' d'ancrage (amorce anti-sens) et 10 nucléotides arbitraires en 5' (amorce sens), en présence de 5 µCi d'[α³²P]-dCTP (Amersham France, Les Ulis, France). Les résultats illustrés à la figure 1 ont été obtenus avec les amorces suivantes : 5'-T₁₁GG (SEQ ID NO:14) et 5'-GTTGCGATCC (SEQ ID NO:15). Les produits PCR ont été dénaturés par la chaleur 3 minutes à 80 °C dans un tampon d'arrêt de séquençage et séparés par électrophorèse en gel dénaturant de poly-acrylamide 6 %. Les gels ont été séchés sans fixation sur du papier filtre Whatman 3MM et exposés pendant 16 heures sur des films Kodak Biomax (Amersham France, Les Ulis, France).

Les bandes qui sont régulièrement apparues comme étant différentielles dans deux amplifications indépendantes, ont été excisées, éluées du gel, réamplifiées dans les mêmes conditions de réaction (35 cycles), analysées sur gel d'agarose 2% et clonées dans un vecteur pCRII (TA Cloning Kit, Invitrogen, Leek, The Netherlands). Un minimum de 40 colonies individuelles recombinantes (blanches) ont été récupérées et la présence des inserts a été testée par PCR avec des amorces flanquantes du vecteur. Plusieurs plasmides recombinants ont été amplifiés par mini-prep, purifiés par kit Qiagen (Qiagen, Courtaboeuf, France) puis radiomarqués avec du [α³²P]-dCTP. Ces plasmides ont servi de sondes pour éliminer les clones similaires. Les clones non redondants ont été séquencés manuellement en utilisant la méthode enzymatique par les di-désoxynucléotides (méthode de Sanger) en présence de Séquanase^{®} 2,0 et des amorces flanquantes du vecteur, en suivant les protocoles du fabricant (US Biochemicals, Cleveland, OH). Les recherches d'homologies de séquences ont été effectuées par l'intermédiaire d'INFOBIOGENBLAST (http://www.infobiogen.fr) sur la GenBank et sur les banques de données de l'EMBL.

### - Isolement de l'ADNc de KLIP-1 (clone n° 36-16) à partir de tissus hématopoïétiques embryonnaires par PCR-RDD

Le profil d'expression du gène pendant l'embryogénèse humaine a été étudié. Les transcrits exprimés de manière différentielle au jour 32 YS (Sac Vitellin), et du jour 27 au jour 49 FL en utilisant la technique de la PCR-RDD ont été examinés. Le clone 36-16 a été isolé du gel RDD et son expression différentielle a été confirmée par la forte expression au jour 25 (sac vitellin), faible expression au jour 32 (sac vitellin), forte expression au jour 28 [AGM (région aorte-gonade-mésonéphros)], comme au jour 28 suivi par la diminution de l'expression aux stades tardifs.

### - Clonage de l'ADNc entier et analyse de la protéine déduite

Une banque d'ADNc de poumon humain (Plasmid MATCHMAKER Library, Clontech, Paris, France) dans le vecteur pACT2 a été utilisée. Les clones d'ADNc 36-16 ont été obtenus par criblage de 1x10⁶ colonies de la banque d'ADNc, en utilisant un insert de 36-16 de 255 pb marqué au ³²P comme sonde pour l'hybridation sur des membranes Hybond N+Nylon. Les clones positifs ont été testés par PCR avec les amorces flanquantes du vecteur et ont été confirmés par hybridation avec l'insert de 36-16 radiomarqué. Deux clones ont présenté un signal d'hybridation positif et une taille appropriée d'environ 1600 pb, en accord avec les résultats du Northem blot. L'un d'eux a été séquencé par la méthode de Sanger, mettant en oeuvre des terminateurs fluorescents et un séquenceur ABI Applied Biosystems (Pharmacia, Orsay, France).

### - Caractérisation de l'ADNc entier de KLIP-1 (36-16) et protéine déduite

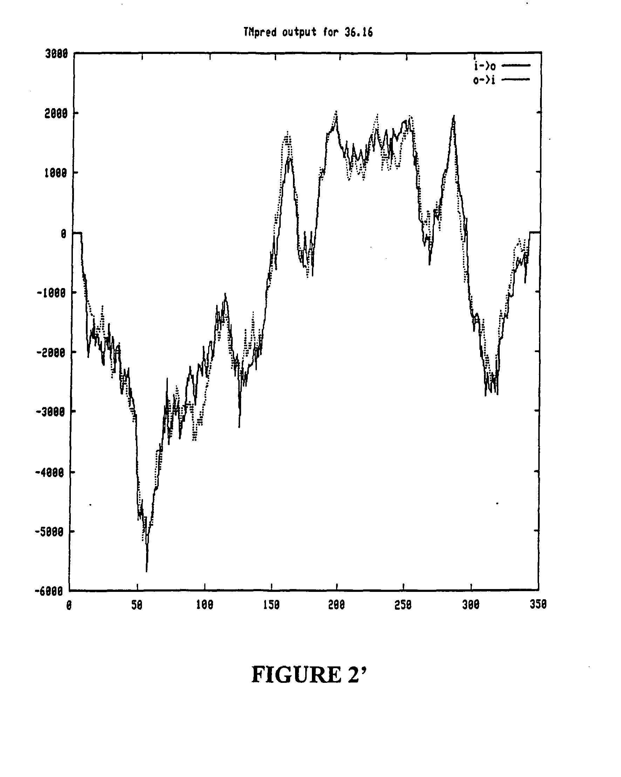

L'ADNc entier comprend 1501 pb et présente un seul cadre ouvert de lecture de 1050 pb avec un UTR 5' de 115 pb et un UTR 3' de 333 pb, ce qui prédit une protéine appelée KLIP-1 de 350 acides aminés avec un poids moléculaire de 38.2 kDa (Figure 2). L'analyse de l'hydrophobicité de la séquence de KLIP-1 suggère 5 hélices TM fortes (Figure 2'). Ainsi, la protéine KLIP-1 présente un peptide signal de l'acide aminé (aa) 1 à 20, un domaine extracellulaire de 132 aa (aa 21 à 152), 5 domaines TM de 143 aa (aa 153-295), et une queue cytoplasmique C-terminale de 54 aa (aa 296 à 350).

### - Cartographie chromosomique RH (Radiation hybrids)

Les clones d'ADNc ont été criblés par PCR, contre le panel d'hybrides irradiés (RH) du génome complet de GeneBridge4 (Research Genetics, Huntsvill, AL), qui contient 93 clones de chimères hamster-humain. Chaque clone a été testé individuellement dans deux amplifications PCR indépendantes avec deux amorces spécifiques de l'UTR 3' : 1265F (5'-ACCCCACCTGAAATTCTTGG) (SEQ ID NO:16) et 1522R (5'-GAGATAAAGAGGAAGGAAGG) (SEQ ID NO:17) dans les conditions suivantes : 94°C 45 sec, 61°C 1 min, 72°C 45 sec pour 10 cycles, puis 94°C 45 sec, 58°C 1 min, 72°C 45 sec pour 30 cycles, terminés par une incubation 10 min à 72°C. Les Southern blots contenant les produits PCR et les ADN contrôles humains (positifs) ou hamster (négatifs) ont été hybridés avec une sonde spécifique interne, marquée au ³²P, et lavés dans des conditions rigoureuses SSC 0,2X + SDS 0,2% à 60°C, puis ont été exposés pendant 16 heures à des films Kodak Biomax. Une seule bande spécifique humaine a été observée à la taille attendue de 257 pb. Les données d'hybridation dérivées du profil de criblage ont été soumises au Whitehead Institute/MIT Centre for Genome Research RH Mapper Server (http://www.genome.wi.mit.edu) et le gène KLIP-1 a été lié à des marqueurs physiques. La carte physique GeneMap'98 (http://www.ncbi.nlm.nih.gov/genemap) a été utilisée pour en déduire la localisation cytogénétique de la bande correspondant aux résultats de la cartographie RH. Afin de confirmer la localisation du gène KLIP-1, des amplifications PCR avec les amorces 1265F (SEQ ID NO:16) et 1522R (SEQ ID NO:17), spécifiques de l'UTR 3' de KLIP-1, hybridées avec la sonde interne 5'-CTGTGGGCACTTCTGAAAGG (SEQ ID NO:18) marquée au ³²P, ont été effectuées sur des clones YAC 907_H_2 et 939_E_12 (Centre d'Etudes du Polymorphisme Humain, Paris, France) contenant respectivement les marqueurs D6S271 et D6S459. Ces marqueurs cernent la région de KLIP-1 sur le chromosome 6 et le marqueur AFM136YD12. L'ADN génomique et le clone YAC 979_B_3, localisé sur le chromosome 1 (D1S444), ont été utilisés respectivement en tant que contrôle positif et négatif.

### - Localisation chromosomique du gène KLIP-1 (36-16) par typage du panel de chimères RH

L'analyse cartographique positionne la séquence de KLIP-1 entre les marqueurs structuraux D6S271 et AFM165YD12 de GeneBridge4, respectivement 5.66 cR de D6S271 et 2.84 cR de AFM65YD 12 à LOD 1.08.

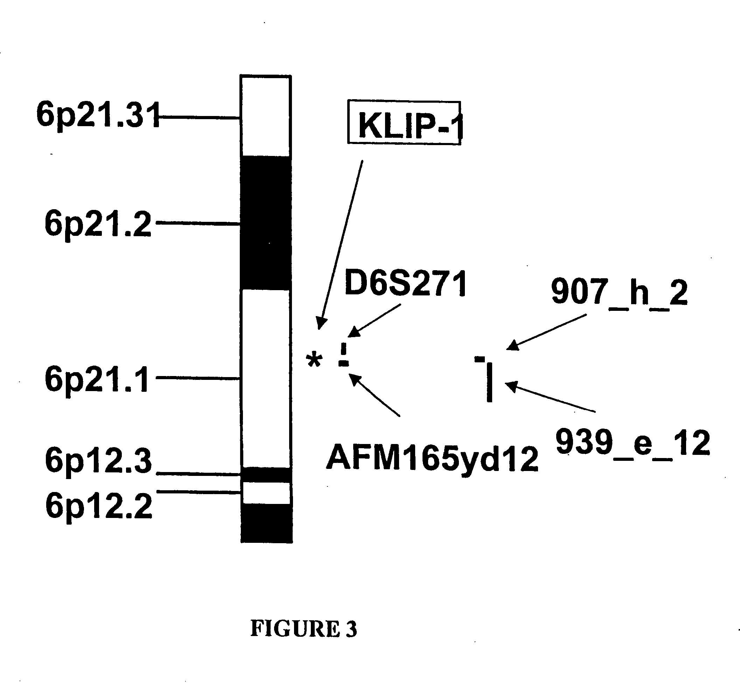

La carte physique GeneMap'98, liée à la localisation de bande cyto-génique, a localisé le gène KLIP-1 en 6p21.1-6p21.2 du chromosome 6. L'amplification PCR, avec les amorces utilisées pour les chimères RH, des clones YACs du CEPH n° 907_h_2 et n°939_e_12, contenant respectivement les marqueurs D6S271 et AFM165YD12, confirme cette localisation. **(****Figure 3****)**

### - Génération de la protéine recombinante KLIP-1

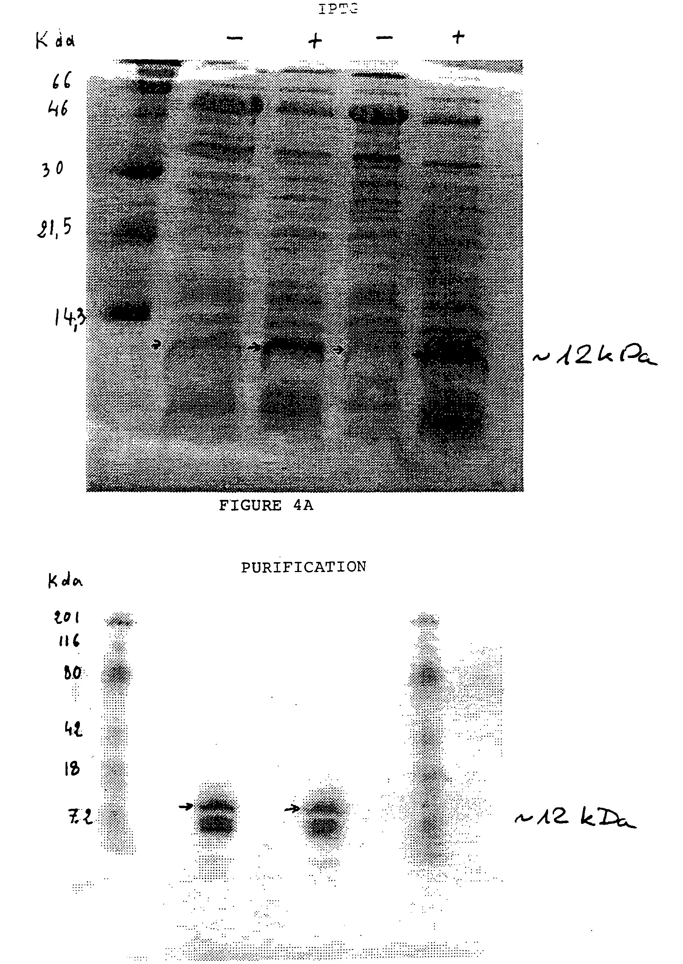

L'ADNc, obtenu après amplification par PCR avec les amorces 180F : 5'-TCGAAGAAAACATCCAGGGC (SEQ ID NO:19) et 556R: 5'-AGAGGTCCATAGAGTTCCACC (SEQ ID NO:20), code pour une protéine extracellulaire de 134 aa des positions 22 à 156. Le produit PCR a été ligué au vecteur d'expression pQE-31 marqué His6X (QIAexpress System, Qiagen, Courtaboeuf, France), puis a été transformé dans la souche M15 portant le plasmide répresseur pREP 4. La construction et le transformant positif ont été criblés par séquençage direct pour une insertion correcte et une conservation en phase. La protéine de 12kDa marquée-His6X, induite à l'IPTG **(****Figure 4A****)**, a été purifiée sur agarose Ni-NTA dans des conditions dénaturantes et a été visualisée par coloration au bleu de Coomassie sur un gel 12% SDS/PAGE **(Figure 4B**).

### - Génération et purification de l'anticorps polyclonal de KLIP-1

Les anticorps (Ab) ont été générés par immunisation mensuelle de lapins White New Zeland avec 100 µl de protéine recombinante extracellulaire de 12 kDa purifiée dans un adjuvant incomplet de Freund. Après 3 injections, le sérum a été collecté puis toutes les IgG ont été purifiées en utilisant les colonnes d'affinité à la protéine G Hitrap (Pharmacia Biotech, Orsay, France) et les anticorps non spécifiques de lapin anti-*E*. *coli* ont été éliminés en utilisant un lysat d'*E. coli* immobilisées (Pierce, Rockford, IL).

### - Révélation par Western Blot

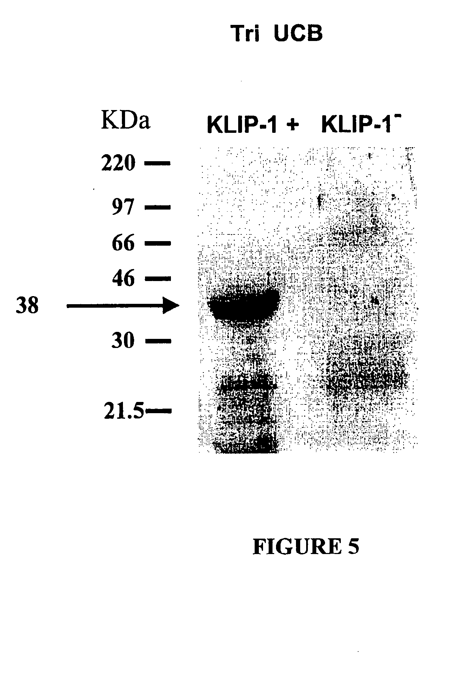

La moelle osseuse totale ou enrichie en cellules KLIP-1+ et les cellules mononucléées du sang du cordon ont été lysées pendant 10 minutes à 100°C dans du tampon contenant Tris (pH 6,8) 50 mM, glycérol 10%, SDS 2%, 2-mercaptoéthanol 5%, et bleu de bromophénol 0,02%. Les protéines (équivalent de 3x10⁵ cellules) ont été séparées par SDS-PAGE 12%, et électrotransférées sur des membranes de nitrocellulose Hybond C-Extra (Amersham, Les Ulis, France). Les membranes ont été bloquées avec du lait en poudre allégé dans du PBS 1X, Tween 20 0,2% pendant 1 heure à température ambiante et ont été incubées avec l'anticorps anti-KLIP-1 1:5000 dans le PBS, Tween 20 0,2%. Après lavage, les blots ont été incubés avec des anticorps de chèvre anti-lapin conjugués à la peroxydase de raifort ; la coloration est obtenue en utilisant un système ECL (Amersham, Les Ulis, France), en accord avec les recommandations du fabricant. Les résultats présentés sur la Figure 5 montrent la présence de la bande de la molécule KLIP à 38 kDa dans la fraction des cellules du sang de cordon KLIP+.

### - Transcription et traduction in vitro de la protéine KLIP-1 et immunoprécipitation.

### 1. Préparation de la matrice

La matrice traduite provient de l'amplification par PCR de l'ADNc du gène KLIP-1 obtenue par Transcription Inverse (MMLV RT, Gibco BRL, France) d'ARN (RNA NOW, Biogentex, France) de cellules mononucléées de sang de cordon. La PCR est effectuée sur un volume total de 100 µl, dans les conditions suivantes :
- 300 nM d'amorce sens +T7
   (5'-AgATCCTAATACGACTCACTATAgggAggAgggACATggCCAACTAAgC-3') (SEQ ID NO:21) et 300 nM anti-sens ou d'amorce inverse (5' AAgAggAAggAAggggTAgg 3') (SEQ ID NO:22).
- Amplification PCR sur 35 cycles (thermocycler 9600, PERKIN ELMER), 94°C 1 minute, 55°C 1 minute, 72°C 2 minutes. Elongation finale 72°C 10 minutes. Le produit d'amplification a été purifié par centrifugation (500 g pendant 20 minutes) sur MICROCON 100 (AMICON, USA), puis repris dans H₂O.

### 2. Transcription et traduction in vitro

La transcription et la traduction de la matrice *in vitro* sont effectuées à partir de :
- 1 µg de matrice
- 40 µl de TnT Quick Maste Mix (Promega Biotech, France)
- 2 µl de ³⁵S méthionine 10 mCi/ml (Amersham, France)
- QSP 50 µl en H₂O
- Les réactifs sont incubés 90 minutes à 30°C.

### 3. Immunoprécipitation

Incubation dans 50 µl de PBS 1X, 5 µl du produit de traduction et 2 µl de l'anticorps polyclonal de lapin anti-KLIP-1, 1 heure à 4°C en agitation.

Ajout de 60 µl de protéine A-sépharose et incubation sur roue 30 minutes à 4°C.

Lavage avec tampon : NP 40 0,1%, Tris-HCl pH 7,5 20 mM, NaCl 150 mM, Na azide 0,05%.

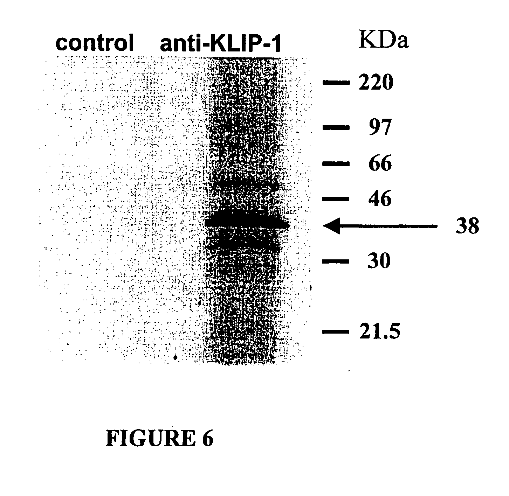

Les échantillons sont analysés par électrophorèse sur un gel 12% SDS-PAGE ; le résultat est visualisé par autoradiographie. La **Figure 6** montre la présence d'une bande correspondant à la molécule KLIP d'environ 38 kDa, détectée par l'anticorps anti-KLIP, alors que le sérum pré-immun témoin ne détecte pas la protéine KLIP. Ceci montre la spécificité de l'anticorps anti-KLIP.

### - Immunosélection de cellules KLIP-1+

Environ 2,5x10⁸ cellules mononucléées du sang périphérique ou du cordon ombilical, de moelle osseuse ou de thymus et du foie foetal, sont incubées dans du sérum humain AB 20% en PBS, 30 minutes à température ambiante. Après lavage en PBS, SVF 2%, les cellules sont incubées avec 90 µl de sérum de lapin anti-KLIP-1(conforme à l'invention) 30 minutes à 4°C, puis incubées avec 50 µl anti-IgG de lapin anti-porc marqué au FITC (Dako, France) 30 minutes à 4°C, puis incubées avec 200 µl de microbilles magnétiques anti-FITC (Miltenyi-Biotech, Paris,France) 30 minutes à 4°C.

Par la suite, les cellules KLIP-1+ sont isolées par des passages successifs sur les colonnes magnétiques LS+ (Miltenyi-Biotech, Paris, France) puis RS+ dans un champ magnétique à l'aide de l'appareil VarioMACS (Miltenyi-Biotech, Paris, France).

Les populations résultantes ont été analysées par FACS-Vantage (Becton Dickinson, France) et la pureté des populations utilisées dans cette étude a varié entre 90 et 99%.

### - Immunophénotypage par FACS

Une analyse par immunofluorescence à 1-, 2-, et 3 couleurs de la population des cellules KLIP-1+ a été effectuée en utilisant un FACS-Vantage (Becton Dickinson, France).

Des fluorochromes conjugués appropriés, sans rapport avec les Igs de contrôle adaptés à l'isotype, ont aussi été utilisés dans toutes les expériences. Les résultats sont présentés par tissus hématopoïétiques :

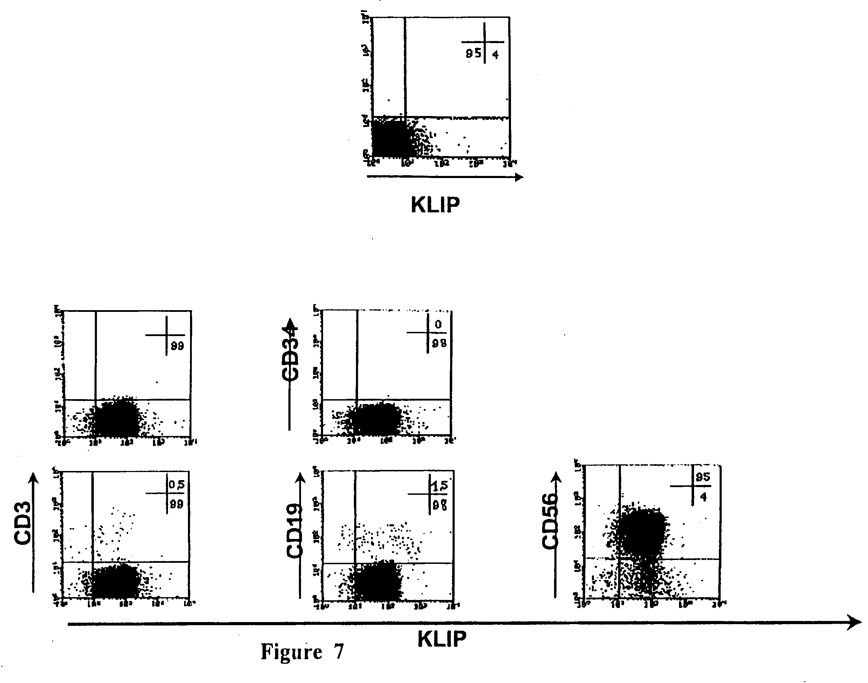

### Figures 7-11 (cellules humaines) :

### Fig. 7: Sang périphérique : contenant uniquement des cellules sanguines matures.

Dans le sang total, 4% des cellules mononucléées sont KLIP-1+ Dans la fraction triée KLIP-1+ avec une pureté de 99%, on trouve 0% CD34+, 95% CD56+ (cellules NK matures), 1,5% CD19+ (cellules B), 0,5% CD3+ (cellules T). Ces deux dernières valeurs étant dues au bruit de fond.

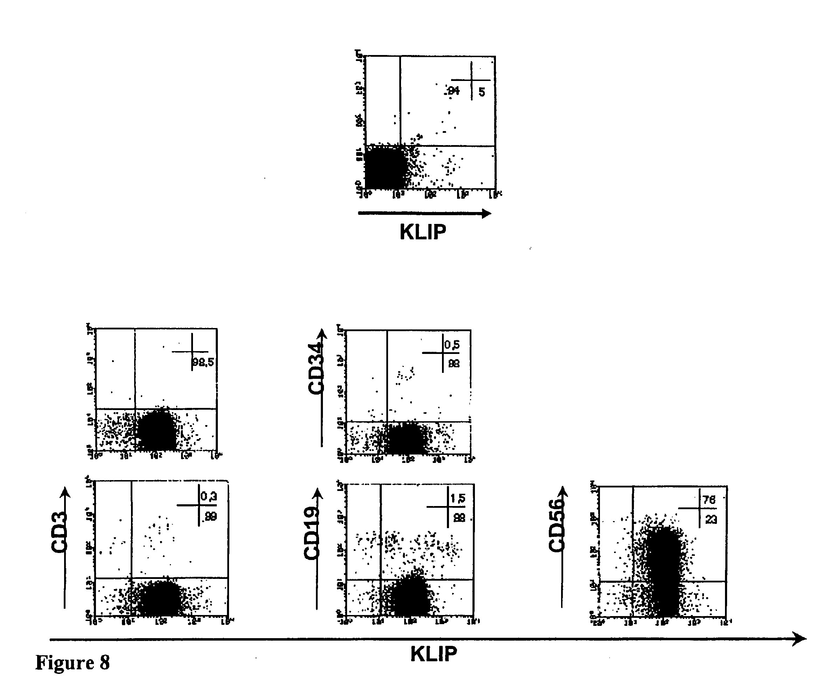

### Fig. 8 : Sang de cordon ombilical, contient en majorité des cellules sanguines matures mais aussi une faible fraction des cellules immatures.

Dans le sang de cordon total, 5% des cellules mononucléées sont KLIP-1+,

Dans la fraction triée KLIP-1+ avec une pureté de 98,5%, on trouve 0,5 % CD34+, 76% CD56+ (cellules NK matures), 1,5% CD19+ (cellules B), 0,3% CD3+ (cellules T).

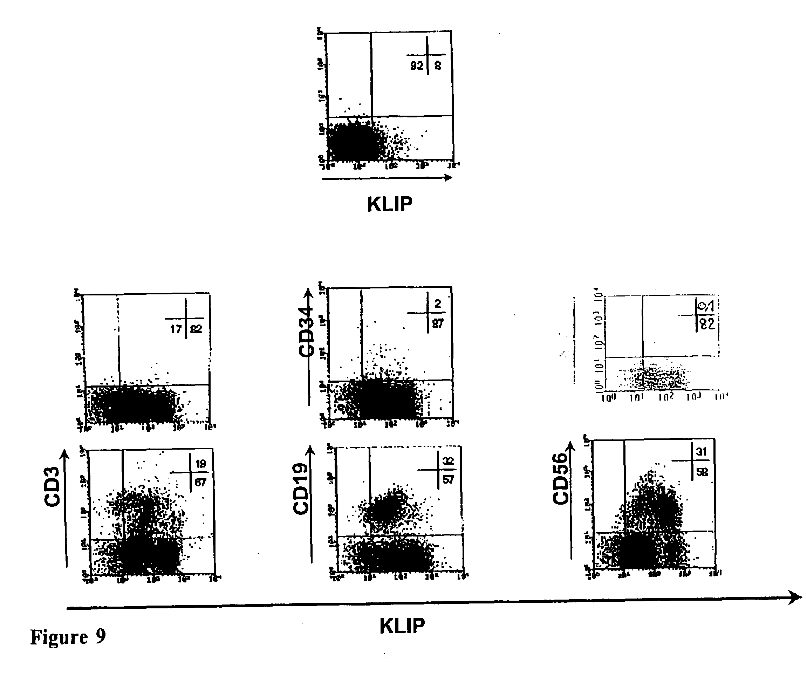

### Fig. 9 : Moelle osseuse : site d'hématopoïèse où s'effectue la différenciation des cellules sanguines ; elle contient donc l'ensemble des cellules sanguines immatures

Dans la moelle osseuse totale, 8% des cellules mononucléées sont KLIP-1+.

Dans la fraction triée KLIP-1+ avec une pureté de 99%, on trouve deux sous-populations « faible intensité et haute intensité » soit 17% KLIP-1 faible + 82% KLIP-1 haute +, et 32% CD19 KLIP-1 faible (cellules B), 19% CD3 KLIP-1 faible (cellules pré-T), 31 % CD56+ KLIP-1 haute + (NK).

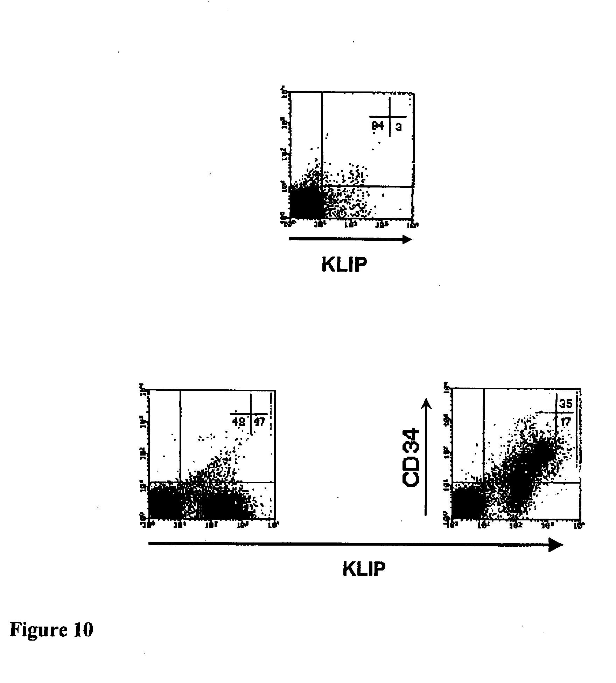

### Fig. 10 : Foie embryonnaire (6 semaines)

Dans les cellules mononucléées du foie total, 3% des cellules sont KLIP-1+.

Dans la mesure où l'on obtient peu de cellules, le rendement a été privilégié par rapport à la pureté.

Des 47% des cellules KLIP-1+, 35% sont CD34+ (progéniteurs hématopoïétiques très précoces).

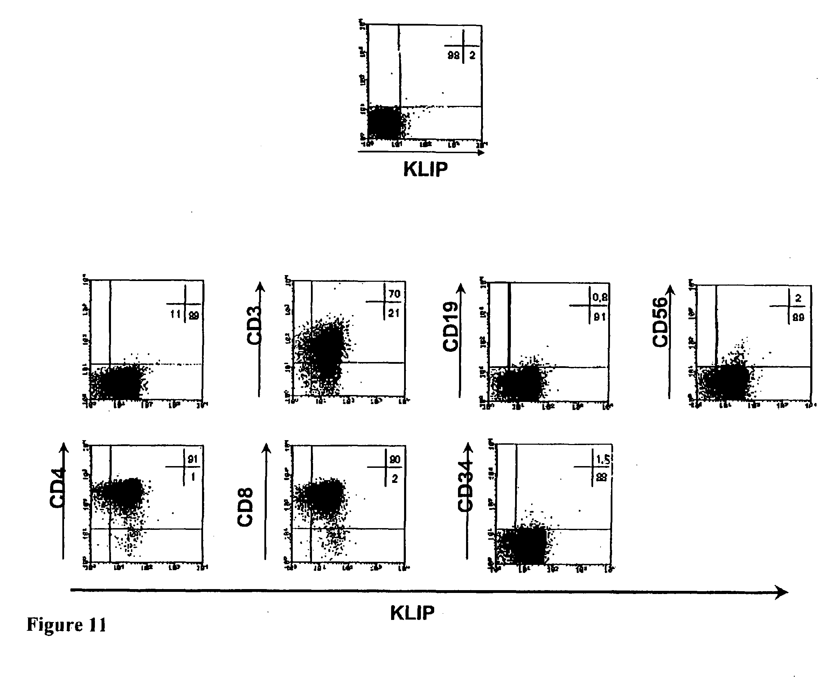

### Fig. 11 : Thymus néo-natal

Le thymus est un organe de maturation immunologique des cellules T.

Dans le thymus total, 2% des cellules mononucléées sont KLIP-1+.

Dans la fraction triée KLIP-1+ avec une pureté de 98%, on trouve 1,5% CD34+, 2% CD56+, 0,8% CD19+ (cellules B), 70% CD3+ (cellules T ou à ce stade de maturation cellules T/NK). Cependant la population KLIP-1+ CD3+ est composée à plus de 91 % des cellules CD4+CD8+ (pré-T).

Ces résultats montrent clairement que le marqueur (antigène ou molécule) KLIP-1 est spécifiquement présent sur les cellules NK tout au long de leur différenciation hématopoïétique, puisqu'il est présent sur les progéniteurs précoces CD34+ de la moelle et de cordon. Il est toujours présent sur les cellules NK immatures CD56- et matures CD56+ et sur les cellules pré-B et pré-T immatures. Dans le thymus néo-natal, la présence de l'antigène KLIP est observée sur les cellules CD3+CD4+CD8+, qui sont des cellules T immatures, car les cellules T matures qui sont CD3+CD4-CD8+ ou CD3+CD4+CD8- sont KLIP-1-.

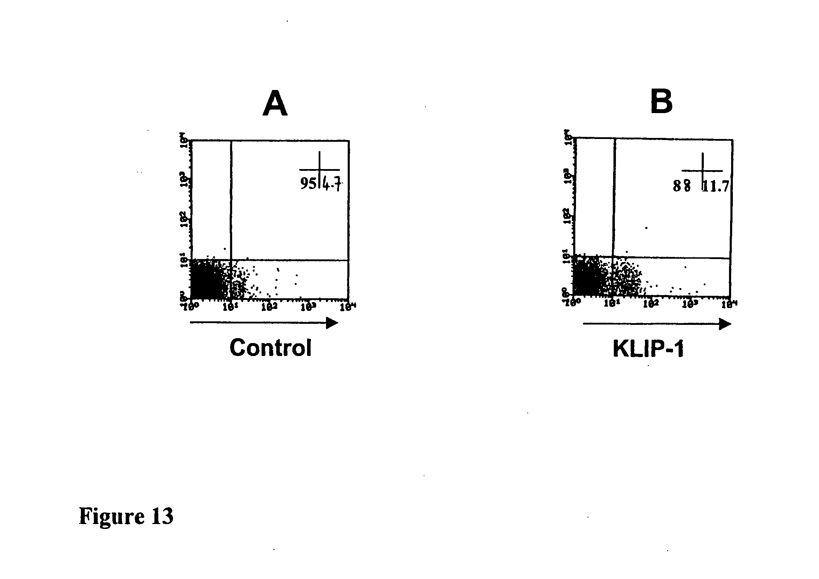

### Figures 13-14 (cellules animales)

Figure 13 (porc) : dans le sang total, 7% des cellules mononucléées sont KLIP-1+ : différence entre le contrôle négatif (figure 13A) obtenu par incubation du sang total de porc avec un sérum de lapin non immunisé et le contrôle positif obtenu avec un sérum de lapin immunisé.

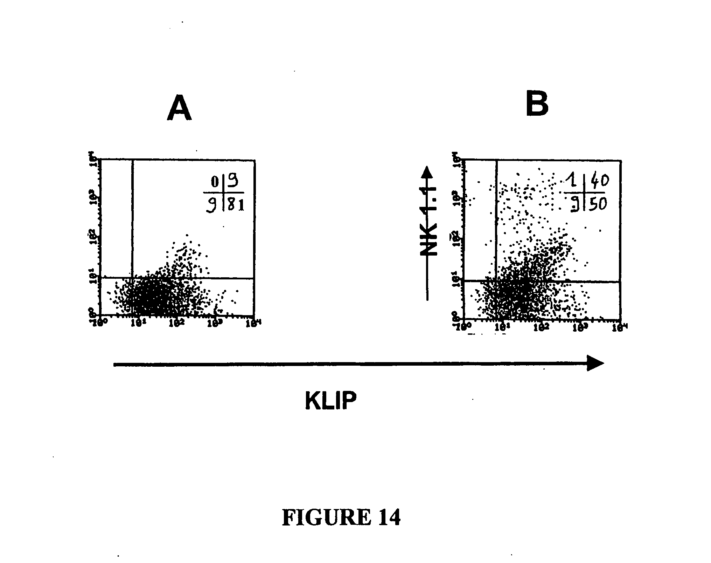

Figure 14 (souris) : figure 14A : dans la fraction triée KLIP-1+ : pureté de 98% (bruit de fond 9 %) ; figure 14B : en présence d'anticorps anti-cellules NK murines (NK1.1), on constate que 40 % des cellules KLIP-1+ sont des cellules NK.

### - Cytotoxicité transmise par les cellules

La cytotoxicité spontanée et la cytotoxicité transmise par cellule dépendante d'anticorps (ADCC) ont été mesurées dans une expérience standard de 4 heures de libération du radioisotope ⁵¹Cr. Des cellules érythroleucémiques K562 sensibles aux NK (American Type Culture Collection, Rockville, MD) ont été utilisées comme cible. Les cellules ciblées ont été marquées avec 200 µCi de chromate⁵¹Cr de sodium (Amersham, Les Ulis, France) pendant 2h. Des cellules effectrices en nombre variable, non mélangées et provenant de différents donneurs (<50 ans) dans 100 µl, ont été ajoutées aux cellules cibles, et incubées pendant 4h à 37°C. Pour mesurer l'ADCC, les cellules ont été préincubées avec le récepteur 1 :50 porc anti-lapin anti-Fc. Toutes les déterminations ont été effectuées en triple.

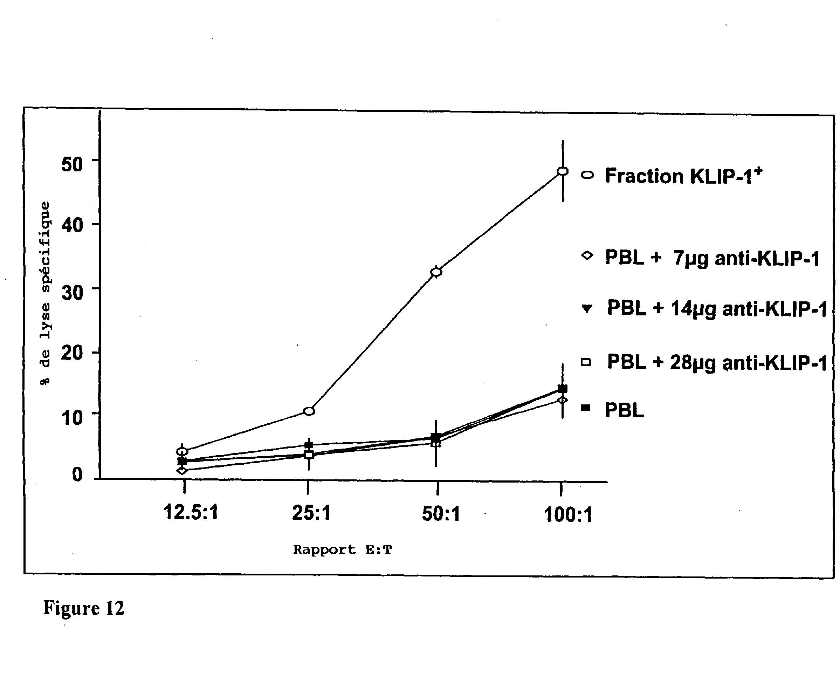

Les résultats obtenus, présentés sur la Figure 12, montrent que :
1. l'anticorps anti-KLIP-1 n'a pas d'effet sur la cytotoxicité des cellules NK, donc l'antigène KLIP n'est ni inhibiteur ni activateur des NK.
2. Le tri (séparation ou sélection) des cellules KLIP-1+ augmente la cytotoxicité probablement par effet de concentration, cela prouve néanmoins que les cellules triées par anti-KLIP-1 dans le sang périphérique sont bien des cellules NK aux fonctions cytotoxiques.

Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## Revendications

1. Protéine membranaire de surface, **caractérisée :**
- **en ce qu**'elle est exprimée à la surface de l'ensemble des cellules progénitrices lymphoïdes et des cellules NK matures,
- en ce qu'elle présente une structure (a) comprenant un domaine extracellulaire situé entre les positions 21 et 152, cinq domaines transmembranaires situés entre les positions 153 et 295 et un domaine cytoplasmique situé entre les positions 296 et 350, en référence à la séquence SEQ ID NO : 2,
- en ce qu'elle présente un poids moléculaire apparent d'environ 36-38 kDa, et
- en ce que son précurseur, qui comprend en N-terminal de la structure en (a) une séquence signal de 20 acides aminés, est sélectionné dans le groupe constitué par la séquence SEQ ID NO : 2 et les variants de cette séquence présentant au moins 70 % d'identité ou au moins 85 % de similarité et de préférence au moins 95 % d'identité ou au moins 99 % de similarité sur la longueur total de ladite séquence SEQ ID NO: 2, à l'exclusion des séquences SEQ ID NO :489 et 606 décrites dans la Demande Internationale PCT WO 98/39448.

2. Protéine selon la revendication 1, **caractérisée en ce qu'**elle est sélectionnée dans le groupe constitué par :
- la protéine humaine de séquence SEQ ID NO: 25, et
- les protéines d'autres mammifères dont le domaine extracellulaire est constitué par les séquences SEQ ID NO:6, 7 ou 24.

3. Fragment de la protéine selon la revendication 1 ou la revendication 2, **caractérisé en ce qu'**il est sélectionné dans le groupe constitué par :
a) un peptide de 7 à 132 acides aminés consécutifs du domaine extracellulaire tel que défini à la revendication 1,
b) un peptide de 7 à 143 acides aminés consécutifs des domaines transmembranaires tels que définis à la revendication 1,
c) un peptide de 7 à 54 acides aminés consécutifs du domaine cytoplasmique tel que défini à la revendication 1, et
d) un peptide de séquence SEQ ID NO : 4 ou 6.

4. Fragment selon la revendication 3, **caractérisé en ce qu'**il est sélectionné dans le groupe constitué par les séquences SEQ ID NO: 7, 8, 9 et 24.

5. Anticorps monoclonal ou polyclonal, **caractérisé en ce qu'**il reconnaît seulement une ou plusieurs protéines selon la revendication 1 ou la revendication 2.

6. Anticorps selon la revendication 5, **caractérisé en ce qu'**il est dirigé contre un peptide du domaine extracellulaire de ladite protéine tel que défini à la revendication 3.

7. Anticorps selon la revendication 6, **caractérisé en ce qu'**il est dirigé contre le peptide de séquence SEQ ID NO : 6, 7 ou 24.

8. ADNc isolé et purifié, **caractérisé en ce qu'**il code pour une protéine ou un fragment de protéine selon l'une quelconque des revendications 1 à 4.

9. ADNc isolé et purifié selon la revendication 8, **caractérisé en ce qu'**il est sélectionné dans le groupe constitué par les séquences SEQ ID NO :1, 3, 5, 10, 11, 12, 13, 16 à 20 et 23.

10. Utilisation d'une protéine selon la revendication 1 ou la revendication 2, comme marqueur de surface des cellules NK matures et/ou des progéniteurs de la lignée lymphoïde.

11. Méthode d'élimination sélective des cellules exprimant le marqueur tel que défini à la revendication 10, à partir d'un échantillon biologique comprenant une population hétérogène de cellules, laquelle méthode comprend :
- la mise en contact dudit échantillon biologique avec un anticorps selon l'une quelconque des revendications 5 à 7, et
- l'élimination des cellules qui se lient audit anticorps.

12. Méthode d'élimination selon la revendication 11, **caractérisée en ce que** ledit anticorps est fixé sur un support solide.

13. Méthode de détection et/ou de quantification et/ou d'isolement des cellules NK et/ou des progéniteurs de la lignée lymphoïde dans une population hétérogène de cellules, laquelle méthode est **caractérisée en ce qu'**elle comprend :
- la mise en contact d'un échantillon comprenant une population hétérogène de cellules avec un anticorps selon l'une quelconque des revendications 5 à 7, et
- la détection de la formation du complexe cellules-anticorps.

14. Méthode selon la revendication 13, **caractérisée en ce que** lorsque ledit échantillon est constitué de moelle osseuse, de sang de cordon ombilical, de foie foetal ou de tout autre tissu hématopoïétique foetal ou adulte, elle peut comprendre en outre la mise en contact des cellules ayant formé un complexe avec ledit anticorps avec un anticorps spécifique des cellules préB tel qu'un anticorps anti-CD19 ou anti-CD 10 et/ou un anticorps spécifique des cellules préT tel qu'un anticorps anti-CD3, anti-CD4 ou anti-CD8.

15. Anticorps selon l'une quelconque des revendications 5 à 7 pour une utilisation comme médicament, notamment dans le traitement des maladies auto-immunes.

16. Anticorps selon l'une quelconque des revendications 5 à 7 pour une utilisation comme réactif de détection et/ou de quantification et/ou d'isolement des cellules NK et/ou des progéniteurs de la lignée lymphoïde.

17. Utilisation des cellules de la moelle osseuse, du sang de cordon ombilical, de foie foetal ou de tout autre tissu hématopoïétique foetal ou adulte, déplétées en cellules NK et en progéniteurs de la lignée lymphoïde, obtenues à l'aide de la méthode selon la revendication 11 ou la revendication 12, pour la préparation d'un produit apte à être transplanté.

18. Méthode de diagnostic des hémopathies malignes et bénignes, **caractérisée en ce qu'**elle comprend la détection dans un échantillon biologique d'une séquence nucléique correspondant à l'ADNc selon la revendication 8 ou la revendication 9.

19. Trousse de détection et/ou de quantification et/ou d'isolement des cellules NK et/ou des progéniteurs de la lignée lymphoïde, **caractérisée en ce qu'**elle comprend outre des quantités utiles de tampons appropriés pour la mise en oeuvre de ladite détection, des doses appropriées d'un anticorps selon l'une quelconque des revendications 5 à 7, éventuellement conjugué à un support solide immunoadsorbant, et des doses appropriées d'un réactif de révélation du complexe cellules-anticorps éventuellement formé.

## Claims

1. Surface membrane protein, **characterized:**
- **in that** it is expressed on the surface of all lymphoid progenitor cells and mature NK cells,
- **in that** it has a structure (a) comprising an extracellular domain located between positions 21 and 152, five transmembrane domains located between positions 153 and 295 and a cytoplasmic domain located between positions 296 and 350, with reference to SEQ ID NO: 2,
- **in that** it has an apparent molecular weight of about 36-38 kDa, and
- **in that** its precursor, which comprises at the N terminal of structure (a) a signal sequence of 20 amino acids, is selected from the group consisting in the sequence SEQ ID NO: 2 and variants of this sequence having at least 70 % identity or at least 85 % similarity and preferably at least 95 % identity or at least 99 % similarity over the total length of the said sequence SEQ ID NO: 2, excluding sequences SEQ ID NO: 489 and 606 described in the PCT international application WO 98/39448.

2. Protein according to claim 1, **characterized in that** it is selected from the group consisting in:
- the human protein of sequence SEQ ID NO: 25 and
- proteins of other mammals of which the extracellular domain consists in sequences SEQ ID NO: 6, 7 or 24.

3. Fragment of the protein according to claim 1 or claim 2, **characterized in that** it is selected from the group consisting in:
a) a peptide of 7 to 132 consecutive amino acids of the extracellular domain as defined in claim 1,
b) a peptide of 7 to 143 consecutive amino acids of the transmembrane domains as defined in claim 1,
c) a peptide of 7 to 54 consecutive amino acids of the cytoplasmic domain as defined in claim 1, and
d) a peptide of sequence SEQ ID NO: 4 or 6.

4. Fragment according to claim 3, **characterized in that** it is selected from the group consisting in sequences SEQ ID NO: 7, 8, 9 and 24.

5. Monoclonal or polyclonal antibody, **characterized in that** it recognizes only one or several proteins according to claim 1 or claim 2.

6. Antibody according to claim 5, **characterized in that** it is directed against a peptide of the extracellular domain of the said protein as defined in claim 3.

7. Antibody according to claim 6, **characterized in that** it is directed against the peptide of sequence SEQ ID NO: 6, 7 or 24.

8. Isolated and purified cDNA, **characterized in that** it codes for a protein or a fragment of protein according to any one of claims 1 to 4.

9. Isolated and purified cDNA according to claim 8, **characterized in that** it is selected from the group consisting in sequences SEQ ID NO: 1, 3, 5, 10, 11, 12, 13, 16 to 20 and 23.

10. Use of a protein according to claim 1 or claim 2 as a surface marker of mature NK cells and/or progenitors of the lymphoid line.

11. Method for selective elimination of cells which express the marker as defined in claim 10 from a biological sample comprising a heterogeneous cell population, which method comprises:
- bringing of the said biological sample into contact with an antibody according to any one of claims 5 to 7, and
- elimination of cells which bind to the said antibody.

12. Method of elimination according to claim 11, **characterized in that** the said antibody is fixed to a solid support.

13. Method for the detection and/or quantification and/or isolation of NK cells and/or progenitors of the lymphoid line in a heterogeneous cell population, which method is **characterized in that** it comprises:
- bringing of a sample comprising a heterogeneous cell population into contact with an antibody according to any one of claims 5 to 7, and
- detection of the formation of the cell-antibody complex.

14. Method according to claim 13, **characterized in that** if the said sample consists in bone marrow, umbilical cord blood, foetal liver or any other foetal or adult haematopoietic tissue, it can also comprise bringing of cells which have formed a complex with the said antibody into contact with a specific antibody of preB cells, such as an anti-CD19 or anti-CD10 antibody, and/or a specific antibody of preT cells, such as an anti-CD3, anti-CD4 or anti-CD8 antibody.

15. Antibody according to any one of claims 5 to 7 for use as a medicament, in particular in the treatment of autoimmune diseases.

16. Antibody according to any one of claims 5 to 7 for use as a reagent for detection and/or quantification and/or isolation of NK cells and/or progenitors of the lymphoid line.

17. Use of cells of bone marrow, umbilical cord blood, foetal liver or any other foetal or adult haematopoietic tissue which are depleted in NK cells and in progenitors of the lymphoid line, obtained with the aid of the method according to claim 11 or claim 12, for the preparation of a product capable of being transplanted.

18. Diagnostic method for malignant and benign haemopathies, **characterized in that** it comprises detection in a biological sample of a nucleic sequence corresponding to the cDNA according to claim 8 or claim 9.

19. Kit for the detection and/or quantification and/or isolation of NK cells and/or progenitors of the lymphoid line, **characterized in that** it comprises besides useful amounts of suitable buffers for carrying out the said detection, appropriate doses of an antibody according to any one of claims 5 to 7, optionally conjugated to a solid immunoadsorbent support, and appropriate doses of a reagent for revealing the cell-antibody complex possibly formed.

## Patentansprüche

1. Oberflächenmembranprotein, **dadurch gekennzeichnet,**
- **dass** es an der Oberfläche der Gesamtheit der lymphoiden Progenitorzellen und der reifen NK-Zellen exprimiert wird;
- **dass** es eine Struktur (a) aufweist, die eine extrazelluläre Domäne, die zwischen den Positionen 21 und 152 liegt, fünf Transmembrandomänen, die zwischen den Positionen 153 und 295 liegen, und eine cytoplasmatische Domäne, die zwischen den Positionen 296 und 350 liegt, bezogen auf die Sequenz SEQ ID NO:2, umfasst;
- **dass** es ein scheinbares Molekulargewicht von etwa 36-38 kDa aufweist und
- **dass** sein Vorläufer, der N-terminal der Struktur (a) eine Signalsequenz von 20 Aminosäuren umfasst, aus der Gruppe ausgewählt ist, die aus der Sequenz SEQ ID NO:2 und den Varianten dieser Sequenz, die wenigstens 70% Identität oder wenigstens 85% Ähnlichkeit und vorzugsweise wenigstens 95% Identität oder wenigstens 99% Ähnlichkeit über die gesamte Länge der genannten Sequenz SEQ ID NO:2 aufweisen, mit Ausnahme der Sequenzen SEQ ID NO:489 und 606, die in der internationalen Anmeldung PCT WO 98/39448 beschrieben sind, besteht.

2. Protein gemäß Anspruch 1, **dadurch gekennzeichnet, dass** es ausgewählt ist aus der Gruppe, die aus:
- dem humanen Protein der Sequenz SEQ ID NO:25 und
- den Proteinen von anderen Säugern, deren extrazelluläre Domäne durch die Sequenzen SEQ ID NO:6, 7 oder 24 gebildet wird, besteht.

3. Fragment des Proteins gemäß Anspruch 1 oder Anspruch 2, **dadurch gekennzeichnet dass** es aus der Gruppe ausgewählt ist, die aus:
a) einem Peptid mit 7 bis 132 fortlaufenden Aminosäuren der extrazellulären Domäne, wie sie in Anspruch 1 definiert ist,
b) einem Peptid mit 7 bis 143 fortlaufenden Aminosäuren der Transmembrandomänen, wie sie in Anspruch 1 definiert sind,
c) einem Peptid mit 7 bis 54 fortlaufenden Aminosäuren der cytoplasmatischen Domäne, wie sie in Anspruch 1 definiert ist, und
d) einem Peptid der Sequenz SEQ ID NO:4 oder 6 besteht.

4. Fragment gemäß Anspruch 3, **dadurch gekennzeichnet, dass** es ausgewählt ist aus der Gruppe, die aus den Sequenzen SEQ ID NO:7, 8, 9 und 24 besteht.

5. Monoklonaler oder polyklonaler Antikörper, **dadurch gekennzeichnet, dass** er nur ein Protein oder mehrere Proteine gemäß Anspruch 1 oder Anspruch 2 erkennt.

6. Antikörper gemäß Anspruch 5, **dadurch gekennzeichnet, dass** er gegen ein Peptid der extrazellulären Domäne des Proteins, wie es in Anspruch 3 definiert ist, gerichtet ist.

7. Antikörper gemäß Anspruch 6, **dadurch gekennzeichnet, dass** er gegen das Peptid der Sequenz SEQ ID NO:6, 7 oder 24 gerichtet ist.

8. Isolierte und gereinigte cDNA, **dadurch gekennzeichnet, dass** sie für ein Protein oder ein Proteinfragment gemäß einem der Ansprüche 1 bis 4 codiert.

9. Isolierte und gereinigte cDNA gemäß Anspruch 8, **dadurch gekennzeichnet, dass** sie ausgewählt ist aus der Gruppe, die aus den Sequenzen SEQ ID NO:1, 3, 5, 10, 11, 12, 13, 16 bis 20 und 23 besteht.

10. Verwendung eines Proteins gemäß Anspruch 1 oder Anspruch 2 als Oberflächenmarker der reifen NK-Zellen und/oder der Progenitoren der lymphoiden Linie.

11. Verfahren zur selektiven Eliminierung der Zellen, die den Marker exprimieren, wie er in Anspruch 10 definiert ist, aus einer biologischen Probe, die eine heterogene Population von Zellen umfasst, wobei das Verfahren umfasst:
- Inkontaktbringen der biologischen Probe mit einem Antikörper gemäß einem der Ansprüche 5 bis 7 und
- Eliminierung der Zellen, die sich an den Antikörper binden.

12. Verfahren der Eliminierung gemäß Anspruch 11, **dadurch gekennzeichnet, dass** der Antikörper an einem festen Träger fixiert wird.

13. Verfahren zur Detektion und/oder Quantifizierung und/oder Isolierung der NK-Zellen und/oder der Progenitoren der lymphoiden Linie in einer heterogenen Population von Zellen, wobei das Verfahren **dadurch gekennzeichnet ist, dass** es umfasst:
- Inkontaktbringen einer Probe, die eine heterogene Population von Zellen umfasst, mit einem Antikörper gemäß einem der Ansprüche 5 bis 7 und
- Detektion der Bildung des Komplexes Zellen-Antikörper.

14. Verfahren gemäß Anspruch 13, **dadurch gekennzeichnet, dass** wenn die Probe aus Knochenmark, Nabelschnurblut, fötaler Leber oder jedem anderen hämatopoietischen fötalen oder erwachsenen Gewebe besteht, das Verfahren außerdem Inkontaktbringen der Zellen, die einen Komplex mit dem Antikörper gebildet haben, mit einem Antikörper, der für preB-Zellen spezifisch ist, wie ein Anti-CD19- oder Anti-CD10-Antikörper, und/oder mit einem Antikörper, der für preT-Zellen spezifisch ist, wie ein Anti-CD3-, Anti-CD4- oder Anti-CD8-Antikörper, umfassen kann.

15. Antikörper gemäß einem der Ansprüche 5 bis 7 zur Verwendung als Medikament, insbesondere in der Behandlung von Autoimmunerkrankungen.

16. Antikörper gemäß einem der Ansprüche 5 bis 7 zur Verwendung als Reagens zur Detektion und/oder Quantifizierung und/oder Isolierung der NK-Zellen und/oder der Progenitoren der lymphoiden Linie.

17. Verwendung von Zellen des Knochenmarks, des Nabelschnurbluts, von fötaler Leber oder jedem anderen fötalen oder erwachsenen hämatopoietischen Gewebe, die frei von NK-Zellen und Progenitoren der lymphoiden Linie sind, die mit Hilfe des Verfahrens gemäß Anspruch 11 oder Anspruch 12 erhalten wurden, zur Herstellung eines Produktes, das geeignet ist, transplantiert zu werden.

18. Verfahren zur Diagnose von malignen und benignen Hämopathien, **dadurch gekennzeichnet, dass** es die Detektion einer Nukleotidsequenz, die der cDNA gemäß Anspruch 8 oder Anspruch 9 entspricht, in einer biologischen Probe umfasst.

19. Kit zur Detektion und/oder Quantifizierung und/oder Isolierung von NK-Zellen und/oder Progenitoren der lymphoiden Linie, **dadurch gekennzeichnet, dass** er außer nützlichen Mengen an Puffern, die für die Durchführung der Detektion geeignet sind, geeignete Dosen eines Antikörpers gemäß einem der Ansprüche 5 bis 7, gegebenenfalls an einen festen immunadsorbierenden Träger konjugiert, und geeignete Dosen eines Reagenzes zum Sichtbarmachen des gegebenenfalls gebildeten Komplexes Zellen-Antikörper umfasst.
